(19) Europäisches Patentamt
European Patent Office
Office européen des brevets

(11) EP 3 398 963 B1

(12) **EUROPEAN PATENT SPECIFICATION**

(45) Date of publication and mention of the grant of the patent:
**29.09.2021 Bulletin 2021/39**

(51) Int Cl.:
*C07K 14/705* (2006.01) *A61K 31/7115* (2006.01)

(21) Application number: **17169561.2**

(22) Date of filing: **04.05.2017**

(54) **CHEMICALLY MODIFIED MRNA FOR USE IN THE TREATMENT OF A DISEASE ASSOCIATED WITH THE CFTR GENE**

CHEMISCH MODIFIZIERTE MRNA ZUR VERWENDUNG BEI DER BEHANDLUNG VON KRANKHEITEN IM ZUSAMMENHANG MIT DEM CFTR-GEN

ARNM CHIMIQUEMENT MODIFIÉ POUR UNE UTILISATION DANS LE TRAITEMENT D'UNE MALADIE ASSOCIÉE AU GÈNE CFTR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.11.2018 Bulletin 2018/45**

(73) Proprietor: **Eberhard Karls Universität Tübingen Medizinische Fakultät**
**72074 Tübingen (DE)**

(72) Inventors:
- **Kormann, Michael**
  **71093 Weil im Schönbuch (DE)**
- **Dewerth, Alexander**
  **72072 Tübingen (DE)**
- **Haque, Ashiqul**
  **72076 Tübingen (DE)**
- **Antony, Justin S.**
  **72076 Tübingen (DE)**

(74) Representative: **Witte, Weller & Partner Patentanwälte mbB**
**Postfach 10 54 62**
**70047 Stuttgart (DE)**

(56) References cited:
- **NADINE BANGEL-RULAND ET AL: "Cystic fibrosis transmembrane conductance regulator-mRNA delivery: a novel alternative for cystic fibrosis gene therapy : CFTR-mRNA delivery for the treatment of CF", JOURNAL OF GENE MEDICINE, vol. 15, no. 11-12, 1 November 2013 (2013-11-01), pages 414-426, XP55419527, US ISSN: 1099-498X, DOI: 10.1002/jgm.2748**
- **KOLTE ATUL ET AL: "PEGylated composite nanoparticles of PLGA and polyethylenimine for safe and efficient delivery of pDNA to lungs", INTERNATIONAL JOURNAL OF PHARMACEUTICS, ELSEVIER, AMSTERDAM, NL, vol. 524, no. 1, 6 April 2017 (2017-04-06) , pages 382-396, XP085000594, ISSN: 0378-5173, DOI: 10.1016/J.IJPHARM.2017.03.094**
- **MICHAEL S D KORMANN ET AL: "Expression of therapeutic proteins after delivery of chemically modified mRNA in mice", NATURE BIOTECHNOLOGY, vol. 29, no. 2, 1 February 2011 (2011-02-01), pages 154-157, XP055040839, ISSN: 1087-0156, DOI: 10.1038/nbt.1733**
- **JUSTIN S. ANTONY ET AL: "Modified mRNA as a new therapeutic option for pediatric respiratory diseases and hemoglobinopathies", MOLECULAR AND CELLULAR PEDIATRICS, vol. 2, no. 1, 20 November 2015 (2015-11-20), pages 1-6, XP055419443, DOI: 10.1186/s40348-015-0022-6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 398 963 B1

- HYEWON YOUN ET AL: "Modified mRNA as an alternative to plasmid DNA (pDNA) for transcript replacement and vaccination therapy", EXPERT OPINION ON BIOLOGICAL THERAPY, vol. 15, no. 9, 2 September 2015 (2015-09-02), pages 1337-1348, XP55406240, ASHLEY, LONDON; GB ISSN: 1471-2598, DOI: 10.1517/14712598.2015.1057563
- KIM NAMHO ET AL: "Barriers to inhaled gene therapy of obstructive lung diseases: A review", JOURNAL OF CONTROLLED RELEASE, ELSEVIER, AMSTERDAM, NL, vol. 240, 16 May 2016 (2016-05-16), pages 465-488, XP029759378, ISSN: 0168-3659, DOI: 10.1016/J.JCONREL.2016.05.031

SEQUENCE LISTING

<110> Eberhard-Karls-UniversitÃ¤t TÃ¼bingen - Medizinische FakultÃ¤t

<120> Chemically modified mRNA for use in the treatment of a disease

associated with the CFTR gene

<130> 5402P571EP

<160> 6

<170> BiSSAP 1.3

<210> 1
<211> 4443
<212> DNA
<213> Artificial Sequence

<220>
<223> CDS of hCFTR

<400> 1

```
atgcagaggt cgcctctgga aaaggccagc gttgtctcca aacttttttt cagctggacc    60
agaccaattt tgaggaaagg atacagacag cgcctggaat tgtcagacat ataccaaatc   120
ccttctgttg attctgctga caatctatct gaaaaattgg aaagagaatg ggatagagag   180
ctggcttcaa agaaaaatcc taaactcatt aatgcccttc ggcgatgttt tttctggaga   240
tttatgttct atggaatctt tttatattta ggggaagtca ccaaagcagt acagcctctc   300
ttactgggaa gaatcatagc ttcctatgac ccggataaca aggaggaacg ctctatcgcg   360
atttatctag gcataggctt atgccttctc tttattgtga ggacactgct cctacaccca   420
gccatttttg gccttcatca cattggaatg cagatgagaa tagctatgtt tagtttgatt   480
tataagaaga ctttaaagct gtcaagccgt gttctagata aaataagtat tggacaactt   540
gttagtctcc tttccaacaa cctgaacaaa tttgatgaag gacttgcatt ggcacatttc   600
gtgtggatcg ctcctttgca agtggcactc ctcatggggc taatctggga gttgttacag   660
gcgtctgcct tctgtggact tggtttcctg atagtccttg ccctttttca ggctgggcta   720
gggagaatga tgatgaagta cagagatcag agagctggga agatcagtga aagacttgtg   780
attacctcag aaatgatcga gaacatccaa tctgttaagg catactgctg ggaagaagca   840
atggaaaaaa tgattgaaaa cttaagacaa acagaactga aactgactcg gaaggcagcc   900
tatgtgagat acttcaatag ctcagccttc ttcttctcag ggttctttgt ggtgttttta   960
tctgtgcttc cctatgcact aatcaaagga atcatcctcc ggaaaatatt caccaccatc  1020
tcattctgca ttgttctgcg catggcggtc actcggcaat ttccctgggc tgtacaaaca  1080
tggtatgact ctcttggagc aataaacaaa atacaggatt tcttacaaaa gcaagaatat  1140
aagacattgg aatataactt aacgactaca gaagtagtga tggagaatgt aacagccttc  1200
tgggaggagg gatttgggga attatttgag aaagcaaaac aaaacaataa caatagaaaa  1260
```

acttctaatg gtgatgacag cctcttcttc agtaatttct cacttcttgg tactcctgtc 1320
ctgaaagata ttaatttcaa gatagaaaga ggacagttgt tggcggttgc tggatccact 1380
ggagcaggca agacttcact tctaatgatg attatgggag aactggagcc ttcagagggt 1440
aaaattaagc acagtggaag aatttcattc tgttctcagt tttcctggat tatgcctggc 1500
accattaaag aaaatatcat ctttggtgtt tcctatgatg aatatagata cagaagcgtc 1560
atcaaagcat gccaactaga agaggacatc tccaagtttg cagagaaaga caatatagtt 1620
cttggagaag gtggaatcac actgagtgga ggtcaacgag caagaatttc tttagcaaga 1680
gcagtataca aagatgctga tttgtattta ttagactctc cttttggata cctagatgtt 1740
ttaacagaaa aagaaatatt tgaaagctgt gtctgtaaac tgatggctaa caaaactagg 1800
attttggtca cttctaaaat ggaacattta aagaaagctg acaaaatatt aattttgcat 1860
gaaggtagca gctattttta tgggacattt tcagaactcc aaaatctaca gccagacttt 1920
agctcaaaac tcatgggatg tgattctttc gaccaattta gtgcagaaag aagaaattca 1980
atcctaactg agaccttaca ccgtttctca ttagaaggag atgctcctgt ctcctggaca 2040
gaaacaaaaa aacaatcttt taaacagact ggagagtttg gggaaaaaag gaagaattct 2100
attctcaatc caatcaactc tatacgaaaa ttttccattg tgcaaaagac tcccttacaa 2160
atgaatggca tcgaagagga ttctgatgag cctttagaga gaaggctgtc cttagtacca 2220
gattctgagc agggagaggc gatactgcct cgcatcagcg tgatcagcac tggccccacg 2280
cttcaggcac gaaggaggca gtctgtcctg aacctgatga cacactcagt taaccaaggt 2340
cagaacattc accgaaagac aacagcatcc acacgaaaag tgtcactggc ccctcaggca 2400
aacttgactg aactggatat atattcaaga aggttatctc aagaaactgg cttggaaata 2460
agtgaagaaa ttaacgaaga agacttaaag gagtgctttt ttgatgatat ggagagcata 2520
ccagcagtga ctacatggaa cacatacctt cgatatatta ctgtccacaa gagcttaatt 2580
tttgtgctaa tttggtgctt agtaattttt ctggcagagg tggctgcttc tttggttgtg 2640
ctgtggctcc ttggaaacac tcctcttcaa gacaaaggga atagtactca tagtagaaat 2700
aacagctatg cagtgattat caccagcacc agttcgtatt atgtgtttta catttacgtg 2760
ggagtagccg acactttgct tgctatggga ttcttcagag gtctaccact ggtgcatact 2820
ctaatcacag tgtcgaaaat tttacaccac aaaatgttac attctgttct tcaagcacct 2880
atgtcaaccc tcaacacgtt gaaagcaggt gggattctta atagattctc caaagatata 2940
gcaattttgg atgaccttct gcctcttacc atatttgact tcatccagtt gttattaatt 3000
gtgattggag ctatagcagt tgtcgcagtt ttacaaccct acatctttgt tgcaacagtg 3060
ccagtgatag tggcttttat tatgttgaga gcatatttcc tccaaacctc acagcaactc 3120

```
aaacaactgg aatctgaagg caggagtcca attttcactc atcttgttac aagcttaaaa      3180
ggactatgga cacttcgtgc cttcggacgg cagccttact ttgaaactct gttccacaaa      3240
gctctgaatt tacatactgc caactggttc ttgtacctgt caacactgcg ctggttccaa      3300
atgagaatag aaatgatttt tgtcatcttc ttcattgctg ttaccttcat ttccatttta      3360
acaacaggag aaggagaagg aagagttggt attatcctga ctttagccat gaatatcatg      3420
agtacattgc agtgggctgt aaactccagc atagatgtgg atagcttgat gcgatctgtg      3480
agccgagtct ttaagttcat tgacatgcca acagaaggta aacctaccaa gtcaaccaaa      3540
ccatacaaga atggccaact ctcgaaagtt atgattattg agaattcaca cgtgaagaaa      3600
gatgacatct ggccctcagg gggccaaatg actgtcaaag atctcacagc aaaatacaca      3660
gaaggtggaa atgccatatt agagaacatt tccttctcaa taagtcctgg ccagagggtg      3720
ggcctcttgg gaagaactgg atcagggaag agtactttgt tatcagcttt tttgagacta      3780
ctgaacactg aaggagaaat ccagatcgat ggtgtgtctt gggattcaat aactttgcaa      3840
cagtggagga aagcctttgg agtgatacca cagaaagtat ttattttttc tggaacattt      3900
agaaaaaact tggatcccta tgaacagtgg agtgatcaag aaatatggaa agttgcagat      3960
gaggttgggc tcagatctgt gatagaacag tttcctggga agcttgactt tgtccttgtg      4020
gatgggggct gtgtcctaag ccatggccac aagcagttga tgtgcttggc tagatctgtt      4080
ctcagtaagg cgaagatctt gctgcttgat gaacccagtg ctcatttgga tccagtaaca      4140
taccaaataa ttagaagaac tctaaaacaa gcatttgctg attgcacagt aattctctgt      4200
gaacacagga tagaagcaat gctggaatgc caacaatttt tggtcataga agagaacaaa      4260
gtgcggcagt acgattccat ccagaaactg ctgaacgaga ggagcctctt ccggcaagcc      4320
atcagcccct ccgacagggt gaagctcttt ccccaccgga actcaagcaa gtgcaagtct      4380
aagccccaga ttgctgctct gaaagaggag acagaagaag agatgcaaga tacaaggctt      4440
tag                                                                    4443
```

<210> 2
<211> 4443
<212> RNA
<213> Artificial Sequence

<220>
<223> mRNA sequence of hCFTR

<400> 2

```
uacgucucca gcggagaccu uuuccggucg caacagaggu uugaaaaaaa gucgaccugg        60
ucugguuaaa acuccuuucc uaugucuguc gcggaccuua acagucugua uaugguuuag       120
ggaagacaac uaagacgacu guuagauaga cuuuuuaacc uuucucuuac ccuaucucuc       180
gaccgaaguu ucuuuuuagg auuugaguaa uuacgggaag ccgcuacaaa aaagaccucu       240
```

aaauacaaga uaccuuagaa aaauauaaau ccccuucagu gguuucguca ugucggagag 300
aaugacccuu cuuaguaucg aaggauacug ggccuauugu uccuccuugc gagauagcgc 360
uaaauagauc cguauccgaa uacggaagag aaauaacacu ccugugacga ggauguggu 420
cgguaaaaac cggaaguagu guaaccuuac gucuacucuu aucgauacaa aucaaacuaa 480
auauucuucu gaaauuucga caguucggca caagaucuau uuuauucaua accuguugaa 540
caaucagagg aaagguuguu ggacuuguuu aaacuacuuc cugaacguaa ccguguaaag 600
cacaccuagc gaggaaacgu ucaccgugag gaguaccccg auuagacccu caacaauguc 660
cgcagacgga agacaccuga accaaaggac uaucaggaac gggaaaaagu ccgacccgau 720
cccucuuacu acuacuucau gucucuaguc ucucgacccu ucuagucacu uucugaacac 780
uaauggaguc uuuacuaacu uuuauagguu agacaauucc guaugacgac ccuucuucgu 840
uaccuuuuuu acuaacuuuu gaauucuguu ugucuugacu uugacugagc cuuccgucgg 900
auacacucua ugaaguuauc gagucggaag aagaagaguc ccaagaaaca ccacaaaaau 960
agacacgaag ggauacguga uuaguuuccu uaguaggagg ccuuuuauaa gugguggua g 1020
aguaagacgu aacaagacgc guaccgccag ugagccguua aagggacccg acauguuugu 1080
accauacuga gagaaccucg uuauuuguuu uauguccuaa agaauguuuu cguucuuaua 1140
uucuguaacc uuauauugaa uugcugaugu cuucaucacu accucuuaca uugucggaag 1200
acccuccucc cuaaaccccu uaauaaacuc uuucguuuug uuuuguuauu guuaucuuuu 1260
ugaagauuac cacuacuguc ggagaagaag ucauuaaaga gugaagaacc augaggacag 1320
gacuuucuau aauuaaaguu cuaucuuucu ccugucaaca accgccaacg accuagguga 1380
ccucguccgu ucugaaguga agauuaccac uaauacccuc uugaccucgg aagucuccca 1440
uuuuaauucg ugucaccuuc uuaaaguaag acaagaguca aaaggaccua auacggaccg 1500
ugguaauuuc uuuuauagua gaaaccacaa aggauacuac uuauaucuau gucuucgcag 1560
uaguuucgua cgguugaucu ucuccuguag agguucaaac gucucuuucu guuauaucaa 1620
gaaccucuuc caccuuagug ugacucaccu ccaguugcuc guucuuaaag aaaucguucu 1680
cgucauaugu uucuacgacu aaacauaaau aaucugagag gaaaaccuau ggaucuacaa 1740
aauugucuuu uucuuuauaa acuuucgaca cagacauuug acuaccgauu guuuugaucc 1800
uaaaaccagu gaagauuuua ccuuguaaau uucuuucgac uguuuuauaa uuaaaacgua 1860
cuuccaucgu cgauaaaaau acccuguaaa agucuugagg uuuuagaugu cggucugaaa 1920
ucgaguuuug aguacccuac acuaagaaag cugguuaaau cacgucuuuc uucuuuaagu 1980
uaggauugac ucuggaaugu ggcaaagagu aaucuucuc uacgaggaca gaggaccugu 2040
cuuuguuuuu uuguuagaaa auuugucuga ccucucaaac cccuuuuuuc cuucuuaaga 2100

uaagaguuag guuaguugag auaugcuuuu aaaagguaac acguuuucug agggaauguu 2160
uacuuaccgu agcuucuccu aagacuacuc ggaaaucucu cuuccgacag gaaucauggu 2220
cuaagacucg ucccucuccg cuaugacgga gcguagucgc acuagucgug accggggugc 2280
gaaguccgug cuuccuccgu cagacaggac uuggacuacu gugugaguca auugguucca 2340
gucuuguaag uggcuuucug uugucguagg ugugcuuuuc acagugaccg gggaguccgu 2400
uugaacugac uugaccuaua uauaaguucu uccaauagag uucuuugacc gaaccuuuau 2460
ucacuucuuu aauugcuucu ucugaauuuc cucacgaaaa aacuacuaua ccucucguau 2520
ggucgucacu gauguaccuu guguauggaa gcuauauaau gacagguguu cucgaauuaa 2580
aaacacgauu aaaccacgaa ucauuaaaaa gaccgucucc accgacgaag aaaccaacac 2640
gacaccgagg aaccuuugug aggagaaguu cuguuucccu uaucaugagu aucaucuuua 2700
uugucgauac gucacuaaua guggucgugg ucaagcauaa uacacaaaau guaaaugcac 2760
ccucaucggc ugugaaacga acgauacccu aagaagucuc cagaugguga ccacguauga 2820
gauuaguguc acagcuuuua aaauguggug uuuuacaaug uaagacaaga aguucgugga 2880
uacaguuggg aguugugcaa cuuucgucca cccuaagaau uaucuaagag guuucuauau 2940
cguuaaaacc uacuggaaga cggagaaugg uauaaacuga aguaggucaa caauaauuaa 3000
cacuaaccuc gauaucguca acagcgucaa aauguuggga uguagaaaca acguugucac 3060
ggucacuauc accgaaaaua auacaacucu cguauaaagg agguuuggag ugucguugag 3120
uuuguugacc uuagacuucc guccucaggu uaaaagugag uagaacaaug uucgaauuuu 3180
ccugauaccu gugaagcacg gaagccugcc gucggaauga aacuuugaga caagguguuu 3240
cgagacuuaa auguaugacg guugaccaag aacauggaca guugugacgc gaccaagguu 3300
uacucuuauc uuuacuaaaa acaguagaag aaguaacgac aauggaagua aagguaaaau 3360
uguuguccuc uuccucuucc uucucaacca uaauaggacu gaaaucggua cuuauaguac 3420
ucauguaacg ucacccgaca uuugaggucg uaucuacacc uaucgaacua cgcuagacac 3480
ucggcucaga aauucaagua acuguacggu ugucuuccau uuggaugguu caguugguuu 3540
gguauguucu uaccgguuga gagcuuucaa uacuaauaac ucuuaagugu gcacuucuuu 3600
cuacuguaga ccgggagucc cccgguuuac ugacaguuuc uagagugucg uuuuaugugu 3660
cuuccaccuu uacgguauaa ucucuuguaa aggaagaguu auucaggacc ggucucccac 3720
ccggagaacc cuucuugacc uagucccuuc ucaugaaaca auagucgaaa aaacucugau 3780
gacuugugac uuccucuuua ggucuagcua ccacacagaa cccuaaguua uugaaacguu 3840
gucaccuccu uucggaaacc ucacuauggu gucuuucaua aauaaaaaag accuuguaaa 3900
ucuuuuuuga accuaggaau acuugucacc ucacuaguuc uuuauaccuu ucaacgucua 3960
cuccaacccg agucuagaca cuaucuuguc aaaggacccu ucgaacugaa acaggaacac 4020

cuacccccga cacaggauuc gguaccggug uucgucaacu acacgaaccg aucuagacaa 4080

gagucauucc gcuucuagaa cgacgaacua cuugggucac gaguaaaccu aggucauugu 4140

augguuuauu aaucuucuug agauuuuguu cguaaacgac uaacguguca uuaagagaca 4200

cuuguguccu aucuucguua cgaccuuacg guuguuaaaa accaguaucu ucucuuguuu 4260

cacgccguca ugcuaaggua ggucuuugac gacuugcucu ccucggagaa ggccguucgg 4320

uagucgggga ggcuguccca cuucgagaaa ggggugggccu ugaguucguu cacguucaga 4380

uucgggggucu aacgacgaga cuuucuccuc ugucuucuuc uccacguucu auguuccgaa 4440

auc 4443

<210> 3
<211> 18
<212> DNA
<213> Artificial Sequence

<220>
<223> hCFTR fwd

<400> 3
tgtacggcta caggggaa 18

<210> 4
<211> 18
<212> DNA
<213> Artificial Sequence

<220>
<223> hCFTR rev

<400> 4
gccgataggc agattgta 18

<210> 5
<211> 18
<212> DNA
<213> Artificial Sequence

<220>
<223> optimal determined house-keeping gene 18S rRNA fwd

<400> 5
gggagcctga gaaacggc 18

<210> 6
<211> 21
<212> DNA
<213> Artificial Sequence

<220>
<223> 18S rRNA rev

<400> 6

gacttgccct ccaatggatc c 21

## Description

The present invention relates to a chemically modified mRNA, a nucleic acid molecule encoding the *CFTR* cmRNA, a vector comprising said nucleic acid molecule, a host cell comprising said vector, and to a pharmaceutical composition comprising the *CFTR* cmRNA, nucleic acid molecule or vector.

FIELD OF THE INVENTION

The present invention relates to the field of molecular biology and molecular medicine, more particularly to the treatment a disease associated with the *CFTR* gene.

BACKGROUND OF THE INVENTION

There are various diseases known in the art which are associated with the CF transmembrane conductance regulator (CFTR) gene. Such diseases include cystic fibrosis (CF), congenital absence of the vas deferens (CAVD) and chronic obstructive lung disease (COPD).

Cystic fibrosis (CF), the most common life-limiting genetic disease in Caucasian populations (1/2,500 newborns), affects more than 80,000 people world-wide. It is caused by the presence of mutations in both copies of the underlying disease-conferring *CFTR* gene. Those with a single working copy are carriers and otherwise mostly normal. CFTR is involved in production of sweat, digestive fluids, and mucus. Those mutations result in impaired anion secretion and hyper-absorption of sodium across epithelia. When CFTR is not functional, secretions which are usually thin instead become thick. Chronic lung disease and slow lung degradation is the major factor contributing to not only the mortality and morbidity in CF patients; it also strongly impairs their quality of life.

No cure for cystic fibrosis is known. Lung infections are treated with antibiotics which may be given intravenously, inhaled, or by mouth. Sometimes, the antibiotic azithromycin is used long term. Inhaled hypertonic saline and salbutamol may also be useful. Lung transplantation may be an option if lung function continues to worsen. With currently available therapies which are very expensive, the mean survival is between 30-40 years.

Since the CFTR gene was first cloned in 1989, attempts have been made to correct the mutations at a cellular and genetic level. Gene therapy approaches made it quickly to the clinic aiming to deliver viral CFTR encoded vectors [such as adenoviruses (Ad) or adeno-associated viruses (AAV)] to CF patients. However, none of the clinical studies and current treatments seem to provide sufficient hCFTR expression to prevent the ultimately lethal CF symptoms in the airways of CF patients. Furthermore, repeated administration of viral vectors or DNA lead to the development of unwanted immune reactions, mainly due to viral capsids and vector-encoded proteins.

Congenital absence of the vas deferens (CAVD) is a condition in which the vasa deferentia reproductive organs, fail to form properly prior to birth. It may either be unilateral (CUAVD) or bilateral (CBAVD). The vas deferens connect the sperm-producing testicles to the penis. Therefore, those who are missing both vas deferens are typically able to create sperm but are unable to transport them appropriately. Their semen does not contain sperm, a condition known as azoospermia. There are two main populations of CAVD; the larger group is associated with cystic fibrosis and occurs because of a mutation in the CFTR gene. Mutation of the CFTR gene is found to result in obstructive azoospermia in postpubertal males with cystic fibrosis. Strikingly, CAVD is one of the most consistent features of cystic fibrosis as it affects up to 99% of individuals in this CF patient population. In contrast, acute or persistent respiratory symptoms present in only 51% of total CF patients.

Individuals with CAVD can reproduce with the assistance of modern technology with a combination of testicular sperm extraction and intracytoplasmic sperm injection (ICSI). However, so far no causative therapy for CAVD is available. Also a surgical treatment is not possible.

Chronic obstructive pulmonary disease (COPD) is a type of obstructive lung disease characterized by long-term poor airflow. The main symptoms include shortness of breath and cough with sputum production. COPD is a progressive disease, meaning it typically worsens over time. Even though tobacco smoking is the most common cause of COPD, cases are known where mutations the *CFTR* gene were described as a disease-conferring cause.

Currently available COPD treatments include stopping smoking, vaccinations, respiratory rehabilitation, and often inhaled bronchodilators and steroids. However, therapeutic measures against forms of COPD resulting from a dysfunctional *CFTR* gene are so far not available.

Kormann et al. (2011), Expression of therapeutic proteins after delivery of chemically modified mRNA in mice, Letters to Nature Biotechnology, pages 1-6, describe a therapeutic approach for the treatment of SP-B deficiency where a functional nucleotide modified messenger RNA (mRNA) encoding SP-B is introduced into alveolar cells of the mouse.

Mahiny et al. (2015), In vivo genome editing using nuclease-encoding mRNA corrects SP-B deficiency, Nat. Biotechnology 33(6):584-586, and WO 2015/052133 disclose a nuclease-encoding nucleotide-modified messenger RNA (nec-mRNA) intended to correct a genetic alteration of the surfactant protein B (SP-B).

Bangel-Ruland et al. (2013), Cystic fibrosis transmembrane conductance regulator-mRNA delivery: a novel alternative for cystic fibrosis gene therapy, J. Gene, Med. 15: 414—426, disclose the use of wtCFTR mRNA as a therapeutic approach for cystic fibrosis.

Kolte et al. (2017), PEGylated composite nanoparticles of PLGA and polyethylenimine for safe and efficient delivery of pDNA to lungs, Int. J. Pharm. 524: 382-396, describe the delivery of plasmid DNA associated with nanoparticles to lung tissue.

Against this background it is a problem underlying the invention to provide a new therapeutic approach and tool for the treatment of a disease associated with the *CFTR* gene which is highly effective, causative and implementable in the health system at reasonable costs. Further, with the new approach and tool the disadvantages of the prior art should be avoided or reduced, respectively.

The present invention satisfies these and other needs.

SUMMARY OF THE INVENTION

The present invention as defined in the claims provides a chemically modified mRNA encoding a CF transmembrane conductance regulator or a derivative thereof *(CFTR* cmRNA) for use in the treatment of a subject having or suspected of having a disease associated with the *CFTR* gene, said *CFTR* cmRNA is complexed with a polylactic-co-glycolic acid (PLGA) based nanoparticle.

According to the invention, "chemically modified mRNA" (cmRNA) refers to such an messenger ribonucleic acid, where at least a part of the nucleotides, or nucleosides or nucleobases is modified or changed with respect to their naturally occurring counterparts by the provision or omission of a chemical structure or entity. In this regard the terms "nucleotides" and "nucleosides" are used interchangeably. The chemical modification has the result that the mRNA is more stable and has less immunogenicity. Nucleotide-modified messenger RNA is generally known in the prior art, cf. for example from WO 2011/012316. Examples for chemically-modified nucleotides or nucleosides are pseudouridine ($\Psi$), 5-methylcytidine (m5C), N6-methyladenosine (m6A), 5-methyluridine (m5U) or 2-thiouridine (s2U).

According to the invention "CF transmembrane conductance regulator" (CFTR) refers to a functional membrane protein and chloride channel of vertebrates that is encoded by or derived from the CFTR gene of a vertebrate, e.g. a human or animal being *(hCFTR).* The *CFTR* gene codes for an ABC transporter-class ion channel protein that conducts chloride and thiocyanate ions across epithelial cell membranes. Exemplary nucleotide and amino acid sequences (NCBI Reference Sequence; RefSeq) of CFTR are as follows: mRNA = NM_000492 (human) and NM_021050 (mouse); protein = NP_000483 (human) and NP_066388.1/NP_066388 (mouse). According to the invention the *CFTR* cmRNA may therefore comprise any of the before identified mRNA sequence or a nucleotide sequence encoding any of the before identified proteins.

According to an embodiment of the invention *"CFTR* cmRNA" includes not only a cmRNA encoding the entire *CFTR* gene product or comprising the full or identical coding sequence of the entire *CFTR* gene of a vertebrate but also such a cmRNA encoding a derivative of the *CFTR* gene product. A "derivative of CFTR" according to the invention is a gene product, i.e. a protein or polypeptide which is still a physiologically and/or functionally active membrane protein and an ABC transporter-class ion channel that conducts chloride and thiocyanate ions across epithelial cell membranes. A "derivative of CFTR" still has the biological activity of the wild type CFTR by at least 50%, further preferably by at least 60%, 70%, 80%, 90%, and highly preferably by 100%, as determined by a well-established functional test known to the skilled person. A "derivative of CFTR" may comprise a section of the full length amino acid sequence of CFTR of a vertebrate, but also an amino acid sequence with a sequence homology to the full length amino acid sequence of CFTR of about at least approx. 90%, preferably of at least approx. 95%, 96%, 97%, 98%, 99% as determinable by BLAST; COBALT, or the like.

According to the invention the CFTR cmRNA includes both single- and double-stranded mRNA while, however, single-stranded mRNA is preferred.

According to the invention a "medicament" refers to a pharmaceutical composition wherein the *CFTR* cmRNA or the coding product is considered as the active agent. The *CFTR* cmRNA or the coding product may be the only active agent in the medicament. In an embodiment of the invention additional active agents may be provided. The medicament may include a pharmaceutically formulation comprising a carrier and, optionally, one or more accessory ingredients. Depending on the way of administration the medicament may be present in a form appropriate for a delivery through the air ways of the patient, e.g. as an aerosol or a fine inhalable powder. Pharmaceutical formulations for oral administration can be formulated using pharmaceutically acceptable carriers well known in the art in appropriate and suitable dosages. Such carriers enable the pharmaceuticals to be formulated in unit dosage forms as tablets, pills, powder, dragees, capsules, liquids, lozenges, gels, syrups, slurries, suspensions, etc., suitable for ingestion by the patient. In particular such capsules, tablet or pills are preferred which are acid-resistant and allow the release of the *CFTR* cmRNA in the gastrointestinal tract. This may result in a restoration of the CFTR channels in the intestine and a release of the *CFTR* cmRNA into the blood stream.

The inventors have surprisingly recognized that a chemically modified mRNA/polyribonucleotide encoding the CF transmembrane conductance regulator *(CFTR* cmRNA) is a valuable tool allowing the treatment of a disease or symptom associated with a defective or dysfunctional *CFTR* gene or protein, respectively. Surprisingly, the invention can be successfully used independent of the underlying *CFTR* dysfunction or mutation, respectively.

The inventors were able to demonstrate not only *in vivo* but also *in vivo* in a well-established mouse model of CFTR-associated diseases that the administration of the *CFTR* cmRNA according to the invention, either intratracheally but also intravenously, results in a drastically increase of the most important cellular parameters in comparison with untreated mice. In fact, as exemplified by lung parameters, the parameters are normalized by the treatment with the *CFTR* cmRNA according to the invention. The inventors were also able to demonstrate the functioning of the CFTR protein encoded by the *CFTR* cmRNA in the transfected cells, both *in vitro* and *in vivo.* Importantly, even a repeated administration of the *CFTR* cmRNA according to the invention does not result in an immune response as demonstrated by the inventors in different scientifically well-accepted immunoassays.

This tremendous therapeutic efficiency combined with a high therapeutic safety of the *CFTR* cmRNA according to the invention was far from being expected but highly surprising for a person skilled in the art. These findings impressively make the *CFTR* cmRNA a valuable therapeutic tool for the treatment of *CFTR* gene associated diseases, such as cystic fibrosis (CF), congenital absence of the vas deferens (CAVD), and chronic obstructive lung disease (COPD).

According to the invention said *CFTR* cmRNA is configured for a use in the treatment of a subject having or suspected of having a disease associated with the *CFTR* gene, preferably said disease is selected from the group consisting of: cystic fibrosis (CF), congenital absence of the vas deferens (CAVD) and/or chronic obstructive lung disease (COPD).

This method has the advantage that a medicament is provided which is superior over the currently used medicaments for the indicated diseases, in particular in terms of therapeutic efficiency, safety and, finally, medical expenses. Furthermore, in contrast to the currently available therapies the invention provides a targeted and causal therapy that addresses and solves the basic genetic problem underlying these diseases. A "subject" as used herein includes any animal or human being.

According to the invention the *CFTR* cmRNA is complexed or associated with with a nanoparticle (NP), e.g. a PLGA-based NP.

This measure has the advantage that the absorption of the *CFTR* cmRNA into the cell or transfection efficiency, respectively, is significantly improved, in particular with respect to cells of the lung tissue but also of other tissues. In the sense of the invention "nanoparticle" refers to a particle between approx. 1 and approx. 300 nanometers in size (hydrodynamic diameter), preferably between approx. 50 nm and approx. 250 nm, further preferably between approx. 75 nm and approx. 200 nm, further preferably between approx. 100 nm and approx. 175 nm, and highly preferably between approx. 150 nm and approx. 160 nm. The indicated sizes can be measured by methods generally known by the skilled person, e.g. after the nanoparticles are dispersed for 30 min in physiological saline or phosphate buffer, e.g. PBS. Well-established measurement techniques include transmission electron microscopy (TEM), scanning electron microscopy (SEM), atomic force microscopy (AFM), photon correlation spectroscopy (PCS), nanoparticle surface area monitor (NSAM), condensation particle counter (CPC), differential mobility analyzer, scanning mobility particle sizer (SMPS), nanoparticle tracking analysis (NTA), X-ray diffraction (XRD), aerosol time of flight mass spectroscopy, aerosol particle mass analyzer (APM). A preferred appropriate nanoparticle is based on polylactic-co-glycolic acid (PLGA). PLGA-based nanoparticles are e.g. described in Danhier et al. (2012), PLGA-based nanoparticles: An overview of biomedical applications, Journal of Controlled Release Vol. 161, Issue 2, p. 505-522. The content of this document in herewith incorporated by reference. Another example for an appropriate nanoparticle is lipid GL67/DOPE.

According to a preferred embodiment of the invention the nanoparticle is at least partially coated with chitosan.

This measure has the advantage that the absorbability or respirability is further increased. In addition, chitosan has been proven as being particularly biocompatible resulting in an increase of the tolerance of the *CFTR* cmRNA by a living being.

According to a preferred embodiment of the invention the *CFTR* cmRNA is configured for an intratracheal (i.t.) administration.

This measure has the advantage that the *CFTR* cmRNA is directly administered to the cells of interest in CF or COPD, i.e. the cells comprising the defective *CFTR* gene. The i.t. administration allows a contacting of the target cells directly in the respiratory tract where the CFTR dysfunction is causing damage. An appropriate configuration according to the invention includes an aerosol or an inhalable fine powder. The i.t. administration can e.g. be realized by means of a common microsprayer aerosolizer or a powder insufflator.

According to a preferred embodiment of the invention the *CFTR* cmRNA is configured for an intravenous (i.v.) administration.

The inventors have surprisingly found that not only an i.t. administration but also an i.v. administration of the CFTR cmRNA results in a targeted expression of CFTR in the cells of interest, such as the airways, the vasa deferentia reproductive organs etc., for reasons which are still not perfectly understood. In this embodiment of the invention a route of administration of the *CFTR* cmRNA is used which has proven highly advantageous. Patients affected by a disease associated with the *CFTR* gene, such as CF or COPD, often suffer from a clotted airway which renders an i.t. administration difficult or even impossible. However, the administration into the blood stream is still possible and still results into an expression of the intact or functioning *CFTR* gen in the affected cells of the airways. A configuration for an i.v. administration includes the provision of the *CFTR* cmRNA in liquid form.

According to the invention a configuration of the *CFTR* cmRNA for an intraarterial (i.a.) or intramuscular (i.m.) administration is also encompassed.

According to another embodiment of the invention said CF transmembrane conductance regulator is of human origin (h*CFTR*).

This measure has the advantage that the requirements for the *CFTR* cmRNA to be used as an active agent in human medicine are established. According to the findings of the inventors the results experimentally demonstrated in the CFTR knock-out mouse model ($Cftr^{-/-}$) allows the conclusion that a similar effect can also be achieved in human beings. The use of *hCFTR* cmRNA according to this preferred embodiment thus provides for the precondition for an application of the invention in humans.

According to another embodiment of the invention said chemical modification is a replacement of non-modified nucleotide(s) by chemically-modified nucleotide(s).

This measure not only results in an increase of stability of the *CFTR* cmRNA but also in a significant reduction of immunogenicity when administered into a living being. The inventors were able to demonstrate that the use of chemically nucleotide-modified h*CFTR* cmRNAs lead to no detectable immune responses in the serum of treated experimental mice *in vivo* and remained within natural fluctuations in a highly sensitive whole blood assay. Besides the nucleotide modification also results in an increased stability of the administered mRNA in contrast to non-modified mRNA.

According to a preferred embodiment up to including approx. 100 % of the uridine nucleotides and/or up to including approx. 100 % of the cytidine nucleotides, preferably up to including approx. 70 % of the uridine nucleotides and/or up to including approx. 70 % of the cytidine nucleotides, further preferably up to including approx. 50 % of the uridine nucleotides and/or up to including approx. 50 % of the cytidine nucleotides, further preferably up to including approx. 25 % of the uridine nucleotides and/or up to including approx. 25 % of the cytidine nucleotides, and highly preferably up to including approx. 10 % of the uridine nucleotides and/or up to including approx. 10 % of the cytidine nucleotides are modified, preferably by replacing UTP with pseudo-UTP, further preferably with N1-Pseudo-UTP, and/or with thio-UTP, further preferably with 2-thio-UTP (s2U), and by replacing cytidine with methyl-CTP, further preferably with 5-methyl-CTP (m5C).

A depletion of uridine (U) and the use of chemically-modified nucleotides may result in such a *CFTR* cmRNA which is not immunogenic without the need of HPLC purification. In a large scale waiving of HPLC purification significantly reduces the costs of the product according to the invention.

Further examples of chemically-modified nucleotides or nucleosides include thiouridine, N1-methylpseudouridine, 5-hydroxymethylcytidine, 5-hydroxymethylcytidine, 5-hydroxymethyluridine, 5-methylcytidine, 5-methoxyuridine, 5-methoxycytidine, 5-carboxymethylesteruridine, 5-formylcytidine, 5-carboxycytidine, 5-hydroxycytidine, thienoguanosine, 5-formyluridine. Each of these chemically-modified nucleotides or nucleosides are, independently from and/or in combination with each other, suitable to replace its non-modified counterpart. Preferred combinations are 5-methylcytidine/thiouridine; 5-hydroxymethylcytidine/5-hydroxymethyluridine; 5-methylcytidine/pseudouridine; 5-methoxyuridine/5-methyluridine; 5-hydroxymethylcytidine/N1-methylpseudouridine; 5-methylcytidine/N1-methylpseudo-uridine; 5-methylcytidine/5-carboxymethylesteruridine; 5-methoxycytidine/N1-methylpseudouridine; 5-hydroxymethylcytidine/5-methoxyuridine; 5-methylcytidine/thienoguanosine; 5-methylcytidine/5-formyluridine.

This measure has the advantage that through the prescribed content of nucleotide modifications a *CFTR* cmRNA is provided which is stable *in vivo* and little to zero immunogenic. Even more, the inventors could surprisingly realize that it is sufficient if only up to including about 10 % to approx. 25 % of the non-modified nucleotides or nucleosides are replaced by their modified counterparts. The inventors could provide evidence that also such slightly modified mRNA is stable and little to zero immunogenic. Since the nucleotide modification is complex, this has the advantage that the *CFTR* cmRNA according to the invention, because of the low concentration of nucleotide modifications and the possible circumvention of HPLC purification, can be produced in a cost-saving manner. Besides of reducing costs, the lowering of the portion of modified nucleotides has also the advantage that the efficiency of the translation is increased. This is because very high portions of specifically modified nucleotides significantly interfere with the translation of the modified mRNA. However, with lower portions an optimum translation can be observed.

In another preferred embodiment in the *CFTR* cmRNA according to the invention approx. 25% of UTP was replaced with 2-thio-UTP (s2U) and approx. 25% of CTP was replaced 5-methyl-CTP (m5C)

According to the findings of the inventors such *CFTR* cmRNA provides optimum characteristics with regard to immunogenicity, stability and the cost-benefit-ratio.

Against this background another subject-matter of the present invention relates to a nucleic acid molecule encoding the *CFTR* cmRNA according to the invention.

As used herein, the term "nucleic acid molecule" refers to any nucleic acid containing molecule, including but not limited to DNA or RNA. Such a nucleic acid enables the targeted expression, replication and handling of CFTR or the *CFTR* mRNA. The nucleic acid may comprise DNA, RNA, locked nucleic acid as well as PNA and it may be a hybrid thereof. The nucleic acid molecule of the present invention preferably is a recombinant nucleic acid molecule. It is evident to the person skilled in the art that regulatory sequences may be added to the nucleic acid molecule of the invention encoding the RNA molecule. For example, promoters, transcriptional enhancers and/or sequences which allow for induced expression of the polynucleotide, i.e., the RNA molecule, of the invention may be employed.

Against this background another subject-matter of the present invention relates to a vector comprising said nucleic acid molecule, and a host cell comprising said vector.

The term "vector" as used herein refers to any nucleic acid molecule, preferably a DNA molecule, used as a vehicle to artificially carry said nucleic acid molecule encoding the *CFTR* cmRNA according to the invention into another cell, where it can be replicated and/or expressed. The vector of the present invention may be, e.g., a plasmid, cosmid, virus, bacteriophage or another vector used e.g. conventionally in genetic engineering, and may comprise further genes such as marker genes which allow for the selection of said vector in a suitable host cell and under suitable conditions. Furthermore, the vector of the present invention may, in addition to the sequences of the nucleic acid molecule encoding the RNA molecule of the invention, comprise expression control elements, allowing proper expression of the coding regions in suitable hosts. Such control elements are known to the skilled person and may include a promoter, a splice cassette, translation start codon, translation and insertion site for introducing an insert into the vector. Preferably, the nucleic acid molecule of the invention is operatively linked to said expression control sequences allowing expression in eukaryotic or prokaryotic cells. Accordingly, the present invention relates to a vector comprising the nucleic acid molecule of the present invention, wherein the nucleic acid molecule is operably linked to control sequences that are recognized by a host cell when the eukaryotic and/or prokaryotic (host) cell is transfected with the vector. Furthermore, the vector of the present invention may also be an expression vector. The nucleic acid molecules and vectors of the invention may be designed for direct introduction or for introduction via liposomes, viral vectors (e.g. adenoviral, retroviral), electroporation, ballistic (e.g. gene gun) or other delivery systems into the cell. Additionally, a baculoviral system can be used as eukaryotic expression system for the nucleic acid molecules of the invention.

Thus, the present invention relates to a host transfected or transformed with the vector of the invention or a non-human host carrying the vector of the present invention, i.e. to a host cell or host which is genetically modified with a nucleic acid molecule according to the invention or with a vector comprising such a nucleic acid molecule. The transformation of the host cell with a vector according to the invention can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc. As used herein "host cell" includes any biological cells of bacterial, fungal, animal, human or plant origin capable of receiving and, preferably, replicating and/or expressing the vector or *CFTR* cmRNA or CFTR, respectively. Examples for suitable fungal cells are yeast cells, preferably those of the genus *Saccharomyces* and most preferably those of the species *Saccharomyces cerevisiae.* Suitable animal cells are, for instance, insect cells, vertebrate cells, preferably mammalian cells, such as e.g. HEK293, NSO, CHO,COS-7, MDCK, U2-OSHela, NIH3T3, MOLT-4, Jurkat, PC-12, PC-3, IMR, NT2N, Sk-n-sh, CaSki, C33A. Further suitable cell lines known in the art are obtainable from cell line depositories, like, e.g., the Deutsche Sammlung von Mikroorganismen und Zellkulturen GmbH (DSMZ) or the American Type Culture Collection (ATCC).

The features, characteristics and advantages of the *CFTR* cmRNA according to the invention apply likewise to the nucleic acid, the vector and the host cell according to the invention and *vice versa.*

In another embodiment of the invention the *CFTR* cmRNA, the nucleic acid molecule, and the vector according to the invention are each being present in lyophilized and/or freeze-dried form.

This measure has the advantage that storage and transportation is greatly facilitated as cooling is not necessarily required. The application of the invention in warm areas including many of the developing countries is herewith made accessible.

Another subject-matter of the invention relates to a pharmaceutical composition for the treatment of a disease associated with the *CFTR* gene comprising the *CFTR* cmRNA and/or the nucleic acid molecule and or the vector, and a pharmaceutically acceptable excipient and/or carrier and/or diluents. Said pharmaceutical composition is preferably configured for an intratracheal (i.t.) administration or, preferably, configured for an intravenous (i.v.) administration.

Thus, preferably, the *CFTR* cmRNA of the present invention is included into the pharmaceutical composition in an effective amount. The term "effective amount" refers to an amount sufficient to induce a detectable therapeutic response in the subject to which the pharmaceutical composition is to be administered. In accordance with the above, the content of the *CFTR* cmRNA of the present invention in the pharmaceutical composition is not limited as far as it is useful for treatment as described above, but preferably contains 0.0000001-10% by weight per total composition.

The features, characteristics and advantages of the *CFTR* cmRNA according to the invention apply likewise to the pharmaceutical composition according to the invention and *vice versa.*

Another subject-matter of the disclosure relates to a method for the treatment of a disease associated with the *CFTR* gene, comprising the following steps, 1. providing the pharmaceutical composition according to the invention, and 2. administering said pharmaceutical composition into a living being.

The living being is, in a preferred variant, a mammal such as a dog, cat, pig, cow, sheep, horse, rodent, e.g., rat, mouse, and guinea pig, or a primate, e.g., gorilla, chimpanzee, and human. In a most preferable embodiment, the subject is a human.

The features, characteristics and advantages of the *CFTR* cmRNA according to the invention apply likewise to the method.

The present invention also relates to a kit comprising the *CFTR* cmRNA molecule of the present invention, the nucleic acid molecule of the present invention, the vector of the present invention or the host cell of the present invention. As regards the preferred embodiments, the same applies, mutatis mutandis, as has been set forth above in the context of the *CFTR* cmRNA molecule, nucleic acid molecule, vector or the host cell according to the present invention. Advantageously, the kit of the present invention further comprises, optionally (a) buffer(s), storage solutions and/or remaining reagents or materials required for the conduct of the above and below uses and methods. Furthermore, parts of the kit of the invention can be packaged individually in vials or bottles or in combination in containers or multicontainer units. The kit of the present invention may be advantageously used, *inter alia,* for carrying out the method of the invention, the preparation of the *CFTR* cmRNA molecule of the invention and could be employed in a variety of applications referred herein, e.g., in the uses as outlined above and below. Another component that can be included in the kit is instructions to a person using a kit for its use. The manufacture of the kits follows preferably standard procedures which are known to the person skilled in the art.

The invention is also described and explained in further detail by referring to the following drawings:

Fig. 1 *(c)mRNA-mediated expression and function of hCFTR in vitro.* (**A**) Percentage of hCFTR positive A549 cells and total expression of hCFTR 24 h and 72 h after transfection with 1 μg h*CFTR* pDNA or (chemically modified) h*CFTR* mRNAs. *, $P < 0.05$ versus unmodified h*CFTR* mRNA, §, $P < 0.05$ vs. pDNA. (**B**) Western Blots, semi-quantifying human CFTR in the cell cultures used in (**A**), normalized to GAPDH and put relative to CFTR levels in HBE cells. *, $P < 0.05$ versus CFBE control at 24 h and §, $P < 0.05$ versus CFBE control at 72 h. (**C**) Quenching efficacy of pDNA or mRNA encoded h*CFTR* in A549 cells relative to untransfected controls was measured at 24 h, 48 h and 72 h post-transfection. *, $P < 0.05$ versus untransfected controls. All bar graph data are depicted as the means ± SD and box plots data are represented as the means ± minimum to maximum values.

Fig. 2 *In vivo study plan, expression of modified hCFTR mRNA and hCFTR protein in mouse lungs and immunogenicity in mice and human whole blood.* (**A)** All mouse groups, particles and particle combinations depicted in the study plan (**B**) and utilized in (**C-F**) are color-coded for their treatment schemes, including dosage and application routes. (**C**) Relative amounts of differently modified hCFTR mRNAs in the lungs, applied i.v. or i.t., compared to 40μg $\text{cmRNA}^{\text{h}CFTR}_{\text{s2U}_{0.25}/\text{m5C}_{0.25}}$ i.t. injection; n = 4-7 mice per group. *, P < 0.05 versus 40μg $\text{cmRNA}^{\text{h}CFTR}_{\text{s2U}_{0.25}/\text{m5C}_{0.25}}$. (**D**) ELISA, detecting specifically human CFTR, was performed on lung preparations at day 6 (endpoint); the same n = 4-7 mice per group as in (**C**) were used. *, P < 0.05, **, P < 0.01 versus untreated CFTR knock-out mice. (**E**) Mice were i.v. or i.t. injected with a mix of (c)mRNA and NPs at a 1:10 ratio, and ELISAs were performed post-i.v./i.t.-injection at three different time points. n.d., not detectable. (**F**) 2 ml whole blood, each from three different healthy human donors, were incubated with either R848 (1 mg/ml) or 3.82 pmol pDNA or 7.91 pmol (c)mRNA (providing the same total number of nucleic acid molecules) and NPs at a 1:10 ratio; after 6 h and 24 h the immune response was determined by ELISA in the sera; * and §, P < 0.05 versus control at 6 h and 24 h, respectively. The red dotted lines in (**D-F**) mark the detection limit as specified in the respective ELISA kit. The blue areas in (**D**, **F**) represent the variance of the negative controls which are biological replicates. n.d., not detectable. All bar graph data are depicted as the means ± SD and box plots data are represented as the means ± minimum to maximum values.

Fig. 3 *In vivo lung function measurements in hCFTR mRNA treated CFTR knock-out mice.* All mouse groups utilized in (**B**-**G**) are color-coded for their treatment schemes (**A**), including dosage and application routes. **B**) Functional test of reconstituted CFTR channel compared to *CFTR* knock-out mice (black), positive controls (violet), and percentages relative to positive control; *n* = 4-7 mice per group. *, P < 0.05, **, P < 0.01 versus untreated *CFTR* knock-out mice. Boxes represent the means ± minimum and maximum values. (**C-G**) Precision *in vivo* lung function measurements covering all relevant outcome parameters on mice treated twice and measured 72 hours after the 2nd installment; *n* = 4-7 mice per group. Data represent the means ± SD on resistance, compliance, newtonian resistance, tissue damping and tissue elastance. *, *P* < 0.05, **, *P* < 0.01 versus untreated *CFTR* knock-out mice.

Examples

1. Material and Methods

*mRNA production*

hCFTR was PCR amplified from pcDNA3.hCFTR with the fusion of *KpnI* and *EcoRI* restriction sites and cloned into a polyA-120 containing pVAX1 (pVAX.A120, www.lifetechnologies.com) by sticky-end ligation using the same restriction sites. The coding DNA sequence ("CDS") of hCFTR is depicted in the enclosed sequence listing under SEQ ID No. 1. The corresponding mRNA sequence is shown under SEQ ID No. 2. For control experiments, DsRed reporter protein was sub-cloned into pVAX1.A120 vector from its original vector pDsRed2 (www.clontech.com). For *in vitro* transcription (IVT), the plasmids were linearized downstream of the poly-A tail with *XhoI* (www.neb.com). IVT reaction was carried out using MEGAscript T7 Transcription kit (www.ambion.com) with an anti-reverse CAP analog (ARCA) in the 5' prime end (www.trilink.com). To produce modified mRNAs, the following chemically modified nucleosides were added to the IVT reaction in the indicated ratios: 100% N1-methylpseudo-UTP, 25% 2-thio-UTP and 25%/100%5-methyl-CTP (www.trilink.com). The hCFTR and DsRed mRNA were purified using the MEGAclear kit (www.ambion.com) and analyzed for size and concentration using a Agilent 2100 Bioanalyzer (www.agilent.com).

*Mammalian cell culture and transfection*

Human bronchial epithelial (HBE) and cystic fibrosis epithelial (CFBE) cell lines were maintained in Minimum Essential Medium (MEM, www.biochrom.com) supplemented with 10% (v/v) heat-inactivated Foetal Calf Serum, L-Glutamine (2mM) and Penicillin -Streptomycin (50U/ml). Cells were incubated at 37°C in a humidified atmosphere containing 5% $CO_2$ until they reached 80-90% confluency. Cell lines were washed with cold sterile PBS and detached by trypsin-EDTA. Trypsinisation was stopped by adding MEM medium with serum. Cells were collected and span down at 500 × g for 5 minutes before resuspension in fresh MEM.

One day before transfection 250,000 cells/well/1ml were plated in 12 well plates and grown overnight in MEM without antibiotics. At a confluency of 70-90%, cells were then transfected with 1000 ng mRNA encoding hCFTR using lipofectamine 2000 (www.invitrogen.com) following the manufacturer's instructions and after changing the media to the reduced serum media, Opti-MEM (www.thermofisher.com). After 5 h, cells were washed with PBS and serum-containing MEM was added. Cells were kept for 24 h and 72 h before further analyses.

*Flow cytometry analyses*

All flow cytometry analyses were performed using a Fortessa X-20 (www.bdbioscience.com). For detection of hCFTR protein in HBE and CFBE cell lines, cells were transfected as described above and subsequently prepared for intracellular staining using a Fixation/Permeabilization Solution Kit as directed in the manufacturer's instruction (www.bdbioscience.com). As primary antibody mouse anti-human hCFTR clone 596 (1:500, kindly provided by the cystic fibrosis foundation therapeutics Inc.) has been used. As secondary antibody served Alexa Fluor 488 goat anti-mouse IgG (1:1,000, www.lifetechnologies.com). At least 20,000 gated cells per tube were counted. Data were analyzed with the FlowJo software.

*YFP-based functional assay*

CFTR activity following transient transfection of CFTR mRNAs in A549 cells was determined using the halide-sensitive yellow fluorescent protein YFP-H148Q/I152L (Galietta et al., 2001) as also described previously (Caputo et al., 2009). A549 cells stably expressing the YFP were plated in 96-well microplates
(50,000 cells/well) in 100 μl of antibiotic-free culture medium and, after 6 h, transfected with either plasmids carrying the coding sequence for CFTR or different CFTR mRNAs. For each well, 0.25 or 0.5 μg of mRNA or plasmid DNA and 0.25 μl of Lipofectamine 2000 were pre-mixed in 50 μl of OPTI-MEM (www.invitrogen.com) to generate transfection complexes that were then added to the cells. After 24 hours, the complexes were removed by replacement with fresh culture medium. The CFTR functional assay was carried out 24, 48, or 72 h after transfection. For this purpose, the cells were washed with PBS and incubated for 20 — 30 min with 60 μl PBS containing forskolin (20 μM). After incubation, cells were transferred to a microplate reader (FluoStar Galaxy; www.bmg.labtech.com) for CFTR activity determination. The plate reader was equipped with high-quality excitation (HQ500/20X: 500 10 nm) and emission (HQ535/30M: 535 15 nm) filters for yellow fluorescent protein (www.chroma.com). Each assay consisted of a continuous 14-s fluorescence reading (5 points per second) with 2 s before and 12 s after injection of 165 μl of a modified PBS containing 137 mM NaI instead of NaCl (final NaI concentration in the well: 100 mM). To determine iodide influx rate, the final 11 s of the data for each well were fitted with an exponential function to extrapolate initial slope. After background subtraction, cell fluorescence recordings were normalized for the initial average value measured before addition of $I^-$. For each well, the signal decay in the final 11 s of the data caused by YFP fluorescence quenching was fitted with an exponential function to derive the maximal slope that corresponds to initial influx of $I^-$ into the cells (Galietta et al., 2001). Maximal slopes were converted to rates of variation of intracellular $I^-$ concentration (in mM/s) using the equation:

$$d[I^-]/dt = K_I[d(F/F_0)/dt]$$

where $K_I$, is the affinity constant of YFP for $I^-$ (Galietta et al., 2001), and $F/F_0$ is the ratio of the cell fluorescence at a given time vs. initial fluorescence.

*Whole blood assay*

Blood from three different donors was taken and collected in EDTA collection tubes (www.sarstedt.com). For each treatment group 2ml of EDTA-blood were transferred into 12-well plates and treated accordingly. Blood treated with R-848 (Resiquimod, www.sigmaaldrich.com) was added at a concentration of 1mg/ml. (un-)modified mRNA and pDNA (each 15μg) were complexed to NPs at a ratio of 1:10. Samples were incubated for 6 h and 24 h at 37 °C in a humidified atmosphere containing 5% $CO_2$. At each time point, 1ml of whole blood was transferred into micro tubes containing serum gel (www.sarstedt.com) and spun down at 10.000 × g for 5 min to obtain serum. Sera were stored at -20 °C for further cytokine measurement analyses.

*Animal experiments*

All animal experiments were approved by the local ethics committee and carried out according to the guidelines of the German Law for the Protection of Animals. *Cftr*$^{-/-}$ mice (CFTR$^{tm1Unc}$) were purchased from Jackson Laboratory (www.jax.org) at an age of 6- to 8-weeks and were maintained under standardized specific pathogen-free conditions on a 12 h light-dark cycle. Food, water as well as nesting material were provided ad *libitum.* Prior to injections mice were anesthetized intraperitoneally (i.p.) with a mixture of medetomidine (0.5 mg/kg), midazolam (5 mg/kg) and fentanyl (50 μg/kg). *Cftr*$^{-/-}$ mice received 20 μg and 40μg of hCFTR mRNA encapsulated in chitosan-coated PLGA nanoparticles [Chitosan (83% deacetylated (Protasan UP CL 113) coated PLGA (poly-D,L-lactide-co-glycolide 75:25 (Resomer RG 752H) nanoparticles; short: NPs] by intratracheal (i.t.) spraying (n=4), and intravenous (i.v.) injection (n=4-7) into the tail vein. Mock treated control *Cftr*$^{-/-}$ mice received 20 μg DsRed mRNA complexed to NPs (n=5) by i.t. delivery. For both interventions, mRNA-NPs were administered in a total volume of 200 μl. Mice received two injections on a three day interval (day 0 and day 3). Detailed description of the i.t. procedures explained elsewhere [Mahiny *et al*., 2016]. After 6 days mice were sacrificed for further end point analyses. To asses immune responses to (un-)modified mRNA C57/BL6 mice (n=4 per group) were treated as described for CFTR$^{-/-}$ mice. As positive controls served mice that received *E. coli* mRNA-NPs (20μg) intravenously. C57BL/6 received one injection of 20 μg mRNA complexed to NPs. After 6 h, 24 h and 72 h mice were sacrificed and blood was collected to obtain serum.

*Pulmonary mechanics*

Lung function for each group was evaluated using a FlexiVent® (www.scireq.com). Prior to tracheostomy mice were anaesthetized intraperitoneally as described above. After anaesthesia, a 0.5 cm incision was performed from the rostral to caudal direction. The flap of skin was retracted, the connective tissue was dissected away, and the trachea was exposed. The trachea was then cannulated between the second and third cartilage rings with a blunt-end stub adapter. The mouse was then connected to the FlexiVent® system and respiratory mechanics were measured.

*Salivary assay*

Prior to tracheostomy, anaesthetized mice were injected 50 μl of $10^{-3}$ M acetycholine (Ach) in the cheek to stimulate production of saliva. The fluid was collected via glas capillaries and a chloride assay was performed using the Chloride Assay Kit according to the manufacturer's protocol (www.sigmaaldrich.com). Briefly, saliva was diluted at a ratio of 1:100 with water in a total volume of 50 μl and subsequently 150 μl chloride reagent was added. After 15 min incubation at room temperature in the dark, absorbance was measured at 620 nm using a Ensight Multimode plate reader (www.perkinelmer.com).

*Western blot analysis*

Protein isolated from cell lines were separated on Bolt NuPAGE 4 — 12 % Bis-Tris Plus gels and a Bolt Mini Gel Tank (all from www.lifetechnologies.com). Immunoblotting for hCFTR was performed by standard procedures according to the manufacturer's instructions using the XCell II Mini-Cell and blot modules (www.lifetechnologies.com). After blocking for 1 h at room temperature, primary antibody against hCFTR (clone 596, 1:1,000) or mouse anti-GAPDH (1:5,000, www.scbt.com) was incubated overnight, horseradish peroxidase-conjugated secondary antibodies (1:5000, anti-mouse from www.dianova.com) were incubated for 1 h at room temperature. Blots were processed by using ECL Prime Western Blot Detection Reagents (www.gelifesciences.com). Semiquantitative analysis was performed using the ImageJ software.

*Real-time RT-PCR*

RNA from lung tissue was isolated using the Precellys Tissue RNA kit according to the maufacturer's protocol. Briefly, parts of the lung were homogenized and lysed with tubes of the Precellys Ceramic Kit 1,4/2,8 mm at 5,000 rpm for 20 s in a Precellys Evolution Homogenizer following RNA-isolation (all from www.peqlab.com). Reverse transcription of 50 ng RNA was carried out using iScript cDNA synthesis kit (www.bio-rad.com). Detection of hCFTR was performed by SYBR-Green based quantitative Real-time PCR in 20 μl reactions on a ViiA7 (www.lifetechnologies.com). Reactions were incubated for 10 min at 95 °C, followed by 40 cycles of 15 s at 95 °C and 2 min at 50 °C (annealing and extension), followed by standard melting curve analysis. The following primer pairs were used: hCFTR fwd TGTACGGCTACAGGGGAA (SEQ ID No. 3), hCFTR rev GCCGATAGGCAGATTGTA (SEQ ID No. 4); optimal determined house-keeping gene 18S rRNA fwd GGGAGCCTGAGAAACGGC (SEQ ID No. 5), 18S rRNA rev GACTTGCCCTCCAATGGATCC (SEQ ID No. 6).

*Enzyme-linked immunosorbent assays (ELISAs)*

To detect protein levels of hCFTR in murine lungs, human CFTR ELISA kit was used (www.elabscience.com). Protein was isolated in 600 μl RIPA-buffer and 5 μl protease inhibitor cocktail using the Precellys Ceramic Kit with a bead size of 1,4/2,8 mm (www.sigmaaldrich.com). Tissue was homogenized in a Precellys Evolution Homogenizer at 6,500 rpm for 10 s for a total of three cycles interrupted by a 15 s break between each spin (www.peqlab.com). Subsequently, supernatants were kept on ice and additionally homogenized for 10 times with a 20G needle and incubated for 20 min (www.bdbioscience.com). Lysates were spun down for 20 min at 13,000 × g and 4 °C. Supernatant was collected and stored at -20 °C for further use. Before hCFTR ELISA detection protein concentration was measured using the Pierce BCA protein assay kit (www.thermofisher.com). For each sample an equal amount of 15 μg whole protein lysate was used. For cytokine measurement, blood from mice and donors was taken to obtain serum and tested for IFN-α and TNF-α production as directed in the manufacturer's instructions (www.bdbioscience.com).

*Statistics*

All analyses were performed using the Wilcoxon-Mann-Whitney test with Graphpad Prsim Version 6 (www.graphpad.com). Data are represented as mean ± SD and $P < 0.05$ (two-sided) was considered statistically significant.

2. Results

*(c)mRNA-mediated expression and function of hCFTR in vitro*

To evaluate the influence of chemical nucleoside modification on *hCFTR* mRNA, the inventors first conducted a set of *in vitro* analyses to characterize the efficacy and functionality of hCFTR protein expression. First, the inventors compared the expression profile of plasmid-encoded hCFTR, unmodified *hCFTR* mRNA and two well-defined nucleoside modifications which have been described to exert state-of-the-art stability/expression *in vitro* or in lung-specific cell contexts *in vivo* (**Fig. 1A**). In one case uridine (UTP) and cytidine (CTP) were fully replaced by N1-Pseudo-UTP and 5-Methyl-CTP abbreviated to $cmRNA^{hCFTR}_{N1\Psi_{1.0}/m5C_{1.0}}$ or partly replaced by the incorporation of 25% 2-Thio-UTP and 25% 5-methyl-CTP, respectively, abbreviated to $cmRNA^{hCFTR}_{s2U_{0.25}/m5C_{0.25}}$. Flow cytometry analyses 24 h after transfection of human cystic fibrosis bronchial epithelial (CFBE) cells showed hCFTR positive cells ranging from 15.8% after *hCFTR* pDNA transfection up to 23.7% after unmodifed h*CFTR* mRNA transfection and to 33.6% and 49.6%after $cmRNA^{hCFTR}_{s2U_{0.25}/m5C_{0.25}}$ and $cmRNA^{hCFTR}_{N1\Psi_{1.0}/m5C_{1.0}}$ transfection, respectively (all mRNA based transfections compared to pDNA, $P < 0.05$; **Fig. 1A,** left lower panel panel, black dots).

Combining (in this case multiplying) the respective median fluorescent intensities (MFIs) with the transfection rates, lead to an arbitrary numbered total hCFTR expression ~11.2-fold higher after $cmRNA^{hCFTR}_{N1\Psi_{1.0}/m5C_{1.0}}$ transfection compared to pDNA, a ~5.5-fold higher expression compared to unmodified RNA, and a ~2.5-fold higher expression compared to $cmRNA^{hCFTR}_{s2U_{0.25}/m5C_{0.25}}$ (all mRNA based total expressions compared to pDNA, $P < 0.05$; Fig. 1A, left upper panel). In contrast, after 72 h all three h*CFTR* (c)mRNAs expressed significantly lower compared to *hCFTR* pDNA transfected cells, reflected both in percentage of positive cells and in total hCFTR expression (P < 0.05; **Fig. 1A,** both right panels).

To confirm and substantiate those findings, the inventors performed Western blot analyses of protein lysates taken from transfected CFBE cells at 24 h and 72 h post treatment **(Fig. 1B).** As positive control served protein lysate from untransfected HBE cells, and GAPDH was used to normalize band intensities. At 24 h h*CFTR* pDNA transfected CFBE cells showed an average of 22.8% of the protein expression of h*CFTR* observed in HBE cells, which increased 4.1-fold to 94.0% at 72h (**Fig. 1B**). This drastic increase of hCFTR expression after pDNA transfection goes well in line with the observations in flow cytometry as does the quick onset of hCFTR expression after h*CFTR* (c)mRNA transfection at 24 h **(Fig. 1B).** However, relative to the 24 h time-point, hCFTR expression either remained nearly static (unmodified h*CFTR* mRNA resulted in 33.8% and 34.7% expression at 24 h and 72 h, respectively), decreased ( $cmRNA^{hCFTR}_{s2U_{0.25}/m5C_{0.25}}$ resulted in 45% and dropped to 29.3% hCFTR expression at 24 h and 72 h, respectively) or increased ( $cmRNA^{hCFTR}_{N1\Psi_{1.0}/m5C_{1.0}}$ started at 46.4% and raised 1.4-fold to 63.3% at 24 h and 72 h, respectively). Ultimately, the expression of h*CFTR* mRNA *in vitro* was strongly dependent on its chemical modification, with $cmRNA^{hCFTR}_{N1\Psi_{1.0}/m5C_{1.0}}$ resulting in the most robust hCFTR expression.

To test for proper function of the (c)mRNA-encoded CFTR channel, the inventors utilized CFTR deficient (ΔF508 mutated) A549 cells which stably express halide-sensitive yellow fluorescent protein (YFP-H148Q/I152L). When adding iodine to the cells, the substance will be transported by existing CFTR channels into the cell. In this case the iodine is quenching the YFP, thereby decreasing its fluorescence signal. Thus, the intensity of the fluorescence signal is inversily correlated with hCFTR function. **Fig. 1C** shows the quenching efficacy after transfection of 250 ng h*CFTR* mRNA into those cells, for three different time points normalized to mock transfected cells. In pDNA transfected cells, the quenching efficacy was significantly higher after 48 h and stayed high even after 72 h ($P < 0.05$), while unmodified as well as modified h*CFTR* mRNA transfected cells revealed a single peak quenching at 48 h ($P < 0.05$), which was in line with the expression patterns.

*hCFTR-(c)mRNA and hCFTR protein quantification in lungs after application in vivo*

The inventors tested for the deposition of h*CFTR* (c)mRNA in the lungs after i.t. or i.v. application via qPCR, quantified the h*CFTR* protein expression using ELISA and then evaluated its immunogenicity depending on modification. Accordingly, an experimental setup has been established (**Fig. 2A**) with unambiguous treatment schemes and main outcome parameters (**Fig. 2B**). After i.t. or i.v. injection of differently modified h*CFTR* cmRNA the lungs were isolated 24 h later, homogenized and lysed, followed by RNA-isolation. After reverse transcription h*CFTR* mRNA was detected and quantified using RT-RealTime-qPCR on a ViiA7, normalized to 18S rRNA, strictly following MIQE protocol for RealTime experiments.

In contrast to the *in vitro* data, when 40 µg $cmRNA^{hCFTR}_{s2U_{0.25}/m5C_{0.25}}$ was i.v. injected into the mice, this resulted in a **~3.8-fold** higher lung deposition compared to 40 µg $cmRNA^{hCFTR}_{N1\Psi_{1.0}/m5C_{1.0}}$ **(Fig. 1C).** Intriguingly, the inventors could detect a significant increase on hCFTR protein level, for which we started with a Precellysis Evolution Homogenizer using optimally sized ceramic beads to isolate the RNA effectively yet gently from lung tissue, then using a just recently developed hCFTR ELISA kit and ended with reading the ELISA on an EnSight Plate Reader for a yet unmatched sensitive hCFTR quantification method. These analyses confirmed that mice treated with 40 µg $cmRNA^{hCFTR}_{s2U_{0.25}/m5C_{0.25}}$ i.t. had a highly significant increase in hCFTR in the lungs of treated mice vs control mice **(Fig. 2D)** (*P* < 0.01). To complement that experiment we tested the effects of an increased amount of $cmRNA^{hCFTR}_{s2U_{0.25}/m5C_{0.25}}$ i.t. to 80 µg, which at first glance still seemed to have a low deposition **(Fig. 2C),** but again showed a clear and significant increase of hCFTR protein compared to control mice **(Fig. 2D).**

*hCFTR-(c)mRNA immunogenicity in vivo in mice after i. v. application and ex vivo in an adapted human whole blood assay*

Being in need for a reliable method to detect immune responses that therapeutic mRNAs may exert in a living being, the inventors focused on two different approaches. First, the inventors applied different compounds such as nanoparticles and R-848 and modified or unmodified mRNA i.v. or i.t. to mice to monitor their immune reaction at three different time points. All compounds, mRNAs and application routes are color-coded in **Fig. 2A.** Surprisingly, applying 40 µg unmodified h*CFTR* mRNA or h*CFTR* cmRNA (with any modifications used) did not lead to detectable responses of IFN-α or TNF-α (detected by ELISA) at all three time points (**Fig. 2E**), which are key cytokines in the immune detection to mRNA. Nanoparticles alone (used in all *in vivo* experiments) showed no immune response over the detection limit. However, the control, *E. coli* extract (total RNA) resulted in a significant increase of IFN-α and TNF-α at 6 h (*P* < 0.05) and a trend that IFN-α was still increased at 24 h, while an effect on at 6 h, which was not detecable at 24 h and 72 h (**Fig. 2E**).

A very different and more complex result was achieved when the inventors used an assay based on human whole blood. After incubation of blood from three different, healthy donors with the respective compounds including positive (R-848) and negative controls (blood only, NPs only), cytokine responses were determined via ELISA at two time points, 6 h and 24 h. Interestingly, the negative controls did not raise IFN-α values above detection limit (**Fig. 2F**), while TNF-α is already measureable in all three human blood samples untreated (with a mean ± SD of 227.3 ± 105.4 pg/ml and 291.7 ± 143.2 pg/ml at 6 h and 24 h, respectively) or treated only with NPs (260.3 ± 160.1 pg/ml and 270.0 ± 143.2 at 6 h and 24 h, respectively). That is the reason why the inventors adapted the graphical presentation of **Fig. 2F** as they already did in **Fig. 2D**, using a blue colored area that represents the variance of the negative controls, which are biological replicates. This makes it much easier to evaluate and assess the effects of the positive control and the actual treated samples as if the inventors would only provide the ELISA detection limit (which—in this case—is obviously far below any of the data points). The positive control (R-848) lead to a stark and significant production of both IFN-α (122.7 ± 38.2 pg/ml and 113.7 ± 40.1 pg/ml at 6 h and 24 h, respectively; *P* < 0.05) and TNF-α (1108.7 ± 21.6 pg/ml and 1070.1 ± 48.3 pg/ml at 6 h and 24 h, respectively; *P* < 0.05) **(Fig. 2F).**

Human whole blood transfected with chemically modified h*CFTR* mRNA (i.e. $cmRNA^{hCFTR}_{s2U_{0.25}/m5C_{0.25}}$ and $cmRNA^{hCFTR}_{N1\Psi_{1.0}/m5C_{1.0}}$ ) showed a very similar result in cytokine expression as observed on the blood only and NP only negative controls: the IFN-α levels did not reach the detection limit of the ELISA; TNF-α responses were 236.0 ± 175.0 pg/ml and 286.3 ± 217.5 pg/ml for $cmRNA^{hCFTR}_{s2U_{0.25}/m5C_{0.25}}$ at 6 h and 24 h, respectively and 225.8 ± 142.6 pg/ml and 297.8 ± 241.0 pg/ml for $cmRNA^{hCFTR}_{N1\Psi_{1.0}/m5C_{1.0}}$ at 6 h and 24 h, respectively **(Fig. 2F).**

Only unmodified h*CFTR* mRNA resulted in a significant increase of IFN-α (30.6 ± 3.0 pg/ml and 16.6 ± 3.5 pg/ml at 6 h and 24 h, respectively; *P* < 0.05) while the TNF-α levels were in line with the negative control and cmRNA values (225.8 ± 154.3 pg/ml and 293.7 ± 194.5 pg/ml at 6 h and 24 h, respectively). Together with the significantly lower expression *in vitro* (**Fig. 1A**) and *in vivo* (data not shown), the inventors focused on modified h*CFTR* mRNA as the most efficient and yet safe gene therapeutic tool in the following therapeutic studies.

*Therapeutic effect of* h*CFTR-(c)mRNA in vivo in mice after i.t. and i.v. application*

After setting up a comprehensive analysis of the unmodified and modified mRNAs used, the inventors investigated their therapeutic potential in a mouse model of Cystic Fibrosis. In order to test the efficacy of h*CFTR* cmRNA, *CFTR* knock-out mice had been used in experimental settings in which main parameters and compounds are explained and color-coded in **Fig. 3A**.

First, the inventors performed a functional test in which we measured the saliva chloride concentration in mice using an acetycholine induction prior to collect the saliva with glass capillaries. Compared to *Cftr*$^{-/-}$ mice, the saliva chloride concentration detected in healthy, *Cftr*$^{+/+}$wild-type mice (mean ± SD of 418.8 ± 48.3 ng/μl, defined as 100%, **Fig. 3B)** was highly significantly lower than in the negative controls (1269 ± 83.8 ng/μl)($P$ < 0.01). The treatment of *Cftr*$^{-/-}$ mice with either $cmRNA^{hCFTR}_{s2U_{0.25}/m5C_{0.25}}$ or $cmRNA^{hCFTR}_{N1\Psi_{1.0}/m5C_{1.0}}$ i.t. or i.v. resulted in significantly lower chloride concentrations in their saliva (between 706.7 ± 63.1 and 780.8 ± 78.0; $P$ < 0.05).

The inventors then finally looked for the lung expression using a Flex-iVent for sensitive *in vivo* measurements on the living mouse. Intriguingly we could find that applying 40 μg or 80 μg of $cmRNA^{hCFTR}_{s2U_{0.25}/m5C_{0.25}}$ or $cmRNA^{hCFTR}_{N1\Psi_{1.0}/m5C_{1.0}}$ i.t. or i.v. highly significantly ($P$ < 0.01) decreased the resistance of the mouse lungs up to healthy, wild-type values (**Fig. 3C**) and at the same time increased the compliance highly significantly (P < 0.01) to values of healthy, wild-type mouse lungs and even beyond (**Fig. 3D**).

3. Summary

In the present work, the inventors provide the first *in vivo* studies delivering chemically modified, human *CFTR* mRNA *(*h*CFTR* cmRNA) to the lungs of *Cftr*$^{-/-}$ mice by intravenous (i.v.) and intratracheal (i.t.) administration of modified h*CFTR* mRNA complexed to biocompatible and biodegradable chitosan-coated PLGA nanoparticles (NPs). The inventors demonstrate cmRNA-NP-mediated hCFTR expression in the lungs of *Cftr*$^{-/-}$ mice, leading to significantly reduced chloride secretion and completely restored lung function parameters. The mRNA can be administered repeatedly, without developing immune responses or losing efficacy, presenting *CFTR* cmRNA as a promising therapeutic for the treatment of patients having or suspected of having a disease associated with the *CFTR* gene, independent of the underlying *CFTR* mutation.

Sequences

| | |
|---|---|
| SEQ ID No.1: | Coding DNA sequence ("CDS") of hCFTR |
| SEQ ID No.2: | mRNA sequence of hCFTR |
| SEQ ID No.3: | DNA sequence of hCFTR fwd primer |
| SEQ ID No.4: | DNA sequence of hCFTR rev primer |
| SEQ ID No.5: | DNA sequence of optimal determined house-keeping gene 18S rRNA fwd primer |
| SEQ ID No.6: | DNA sequence of optimal determined house-keeping gene 18S rRNA rev primer |

## Claims

1. Chemically modified mRNA encoding a CF transmembrane conductance regulator or a derivative thereof *(CFTR* cmRNA) for use in the treatment of a subject having or suspected of having a disease associated with the *CFTR* gene, said *CFTR* cmRNA is complexed with a polylactic-co-glycolic acid (PLGA) based nanoparticle.

2. *CFTR* cmRNA for use of claim 1, wherein said disease is selected from the group consisting of: cystic fibrosis (CF), congenital absence of the vas deferens (CAVD) and/or chronic obstructive lung disease (COPD).

3. *CFTR* cmRNA for use of claim 1, wherein said nanoparticle is at least partially coated with chitosan.

4. *CFTR* cmRNA for use of any of the preceding claims configured for an intratracheal (i.t.) administration or an intravenous (i.v.) administration.

5. *CFTR* cmRNA for use of any of the preceding claims, wherein said CF transmembrane conductance regulator is of human origin (h*CFTR*).

6. *CFTR* cmRNA for use of any of the preceding claims, wherein said chemical modification is a replacement of non-modified nucleotides by chemically-modified nucleotides.

7. *CFTR* cmRNA for use of claim 6, wherein up to including 100 % of the uridine nucleotides and/or up to including 100 % of the cytidine nucleotides, preferably up to including 70 % of the uridine nucleotides and/or up to including 70 % of the cytidine nucleotides, further preferably up to including 50 % of the uridine nucleotides and/or up to including 50 % of the cytidine nucleotides, further preferably up to including 25 % of the uridine nucleotides and/or up to including 25 % of the cytidine nucleotides and highly preferably up to including 10 % of the uridine nucleotides and/or up to including 10 % of the cytidine nucleotides are modified, preferably by replacing UTP with pseudo-UTP, further preferably with N1-Pseudo-UTP, and/or with thio-UTP, further preferably with 2-thio-UTP (s2U), and by replacing cytidine with methyl-CTP, further preferably with 5-methyl-CTP (m5C).

8. *CFTR* cmRNA for use of claim 7, wherein 25% of CTP was replaced with 2-thio-UTP (s2U) and 25% of CTP was replaced 5-methly-CTP (m5C) re-sulting in $\mathrm{cmRNA}^{\mathrm{h}\mathit{CFTR}}_{\mathrm{s2U}_{0.25}/\mathrm{m5C}_{0.25}}$.

9. Chemically modified mRNA encoding a CF transmembrane conductance regulator or a derivative thereof *(CFTR* cmRNA) for the treatment of a subject having or suspected of having a disease associated with the *CFTR* gene, said *CFTR* cmRNA is complexed with a polylactic-co-glycolic acid (PLGA) based nanoparticle.

10. Pharmaceutical composition for the treatment of a disease associated with the *CFTR* gene comprising the *CFTR* cmRNA of any of claims 1-9, and a pharmaceutically acceptable excipient and/or carrier and/or diluents, said CFTR cmRNA is complexed with a polylactic-co-glycolic acid (PLGA) based nanoparticle.

## Patentansprüche

1. Chemisch modifizierte mRNA, die für einen CF-Transmembranleitfähigkeitsregulator oder ein Derivat davon *(CFTR* cmRNA) codiert, zur Verwendung bei der Behandlung eines Patienten, der eine mit dem CFTR-Gen assoziierte Krankheit hat oder bei dem der Verdacht auf eine solche Krankheit besteht, wobei die *CFTR* cmRNA mit einem Nanopartikel auf der Basis von Poly(lactid-co-glycolid) (PLGA) komplexiert ist.

2. *CFTR* cmRNA zur Verwendung nach Anspruch 1, wobei die Krankheit ausgewählt ist aus der Gruppe bestehend aus: zystischer Fibrose (CF), kongenitaler Abwesenheit des Vas deferens (CAVD) und/oder chronisch obstruktiver Lungenerkrankung (COPD).

3. *CFTR* cmRNA zur Verwendung nach Anspruch 1, wobei das Nanopartikel zumindest teilweise mit Chitosan beschichtet ist.

4. *CFTR* cmRNA zur Verwendung nach einem der vorhergehenden Ansprüche, die für eine intratracheale (i.t.) Verabreichung oder eine intravenöse (i.v.) Verabreichung ausgebildet ist.

5. *CFTR* cmRNA zur Verwendung nach einem der vorhergehenden Ansprüche, wobei der CF-Transmembranleitfähigkeitsregulator menschlichen Ursprungs ist (h*CFTR).*

6. *CFTR* cmRNA zur Verwendung nach einem der vorhergehenden Ansprüche, wobei die chemische Modifikation ein Ersatz von nicht-modifizierten Nukleotiden durch chemisch modifizierte Nukleotide ist.

7. *CFTR* cmRNA zur Verwendung nach Anspruch 6, wobei bis zu einschließlich 100 % der Uridin-Nukleotide und/oder bis zu einschließlich 100 % der Cytidin-Nukleotide, vorzugsweise bis zu einschließlich 70 % der Uridin-Nukleotide und/oder bis zu einschließlich 70 % der Cytidin-Nukleotide, weiter vorzugsweise bis zu einschließlich 50 % der Uridin-Nukleotide und/oder bis zu einschließlich 50 % der Cytidin-Nukleotide, weiter bevorzugt bis zu einschließlich 25 % der Uridin-Nukleotide und/oder bis zu einschließlich 25 % der Cytidin-Nukleotide, und ganz besonders bevorzugt bis zu einschließlich 10 % der Uridin-Nukleotide und/oder bis zu einschließlich 10 % der Cytidin-Nukleotide modifiziert sind, vorzugsweise durch Ersetzen von UTP durch Pseudo-UTP, weiter bevorzugt durch N1-Pseudo-UTP, und/oder durch Thio-UTP, weiter bevorzugt durch 2-Thio-UTP (s2U), und durch Ersetzen von Cytidin durch Methyl-CTP, weiter bevorzugt durch 5-Methyl-CTP (m5C).

8. *CFTR* cmRNA zur Verwendung nach Anspruch 7, wobei 25% des CTP durch 2-Thio-UTP (s2U) und 25% des CTP durch 5-Methly-CTP (m5C) ersetzt wurden, was zu $cmRNA^{hCFTR}_{s2U_{0.25}/m5C_{0.25}}$ führt.

9. Chemisch modifizierte mRNA, die für einen CF-Transmembranleitfähigkeitsregulator oder ein Derivat davon codiert *(CFTR* cmRNA), zur Behandlung eines Patienten, der eine mit dem CFTR-Gen assoziierte Krankheit hat oder bei dem der Verdacht auf eine solche Krankheit besteht, wobei die *CFTR* cmRNA mit einem Nanopartikel auf der Basis von Poly(lactid-co-glycolid) (PLGA) komplexiert ist.

10. Pharmazeutische Zusammensetzung zur Behandlung einer mit dem CFTR-Gen assoziierten Krankheit, die die CFTR-cmRNA nach einem der Ansprüche 1 bis 9 und einen pharmazeutisch akzeptablen Hilfsstoff und/oder Träger und/oder Verdünnungsmittel aufweist, wobei die *CFTR* cmRNA mit einem Nanopartikel auf der Basis von Poly(lactid-co-glycolid) (PLGA) komplexiert ist.

**Revendications**

1. ARNm chimiquement modifié codant pour un régulateur de conductance transmembranaire de la fibrose kystique (CF) ou un dérivé de celui-ci (ARNmc de *CFTR)* pour une utilisation dans le traitement d'un sujet ayant ou suspecté d'avoir une maladie associée au gène *CFTR,* ledit ARNmc de *CFTR* est complexé avec une nanoparticule à base d'acide polylactique-co-glycolique (PLGA).

2. ARNmc de *CFTR* pour une utilisation selon la revendication 1,
dans lequel ladite maladie est choisie dans le groupe constitué par : la fibrose kystique (CF), l'absence congénitale du canal déférent (CAVD) et/ou la maladie pulmonaire obstructive chronique (MPOC).

3. ARNmc de *CFTR* pour une utilisation selon la revendication 1, dans lequel ladite nanoparticule est au moins partiellement revêtue de chitosane.

4. ARNmc de *CFTR* pour une utilisation selon l'une des revendications précédentes configuré pour une administration intratrachéale (i.t.) ou une administration intraveineuse (i.v.).

5. ARNmc de *CFTR* pour une utilisation selon l'une des revendications précédentes, dans lequel ledit régulateur de conductance transmembranaire de CF est d'origine humaine *(hCFTR).*

6. ARNmc de *CFTR* pour une utilisation selon l'une des revendications précédentes, dans lequel ladite modification chimique est un remplacement de nucléotides non modifiés par des nucléotides chimiquement modifiés.

**7.** ARNmc de *CFTR* pour une utilisation selon la revendication 6, dans lequel jusqu'à 100% des nucléotides d'uridine et/ou jusqu'à 100% des nucléotides de cytidine, de préférence jusqu'à 70% inclus des nucléotides d'uridine et/ou jusqu'à 70% inclus des nucléotides de cytidine, en outre de préférence jusqu'à 50% inclus des nucléotides d'uridine et/ou jusqu'à 50% inclus des nucléotides de cytidine, en outre de préférence jusqu'à 25% inclus des nucléotides d'uridine et/ou jusqu'à 25% inclus des nucléotides de cytidine et mieux encore jusqu'à 10% inclus des nucléotides d'uridine et/ou jusqu'à 10% inclus des nucléotides de cytidine sont modifiés, de préférence en remplaçant l'UTP par un pseudo-UTP, en outre de préférence par le N1-Pseudo-UTP, et/ ou par le thio-UTP, en outre de préférence par le 2-thio-UTP (s2U), et en remplaçant la cytidine par le méthyl-CTP, en outre de préférence par le 5-méthyl-CTP (m5C).

**8.** ARNmc de *CFTR* pour une utilisation selon la revendication 7, dans lequel 25% de CTP ont été remplacés par du 2-thio-UTP (s2U) et 25% de CTP ont été remplacés par du 5-méthly-CTP (m5C) donnant lieu à un

$$\text{ARNmc}^{\text{h}\mathit{CFTR}}_{\text{s2U}_{0,25}/\text{m5C}_{0,25}}.$$

**9.** ARNm chimiquement modifié codant pour un régulateur de conductance transmembranaire de CF ou un dérivé de celui-ci (ARNmc de *CFTR)* pour le traitement d'un sujet ayant ou suspecté d'avoir une maladie associée au gène *CFTR,* ledit ARNmc de *CFTR* est complexé avec une nanoparticule à base d'acide polylactique-co-glycolique (PLGA).

**10.** Composition pharmaceutique pour le traitement d'une maladie associée au gène *CFTR* comprenant l'ARNmc de *CFTR* de l'une des revendications 1 à 9, et un excipient et/ou un support et/ou des diluants pharmaceutiquement acceptable(s), ledit ARNmc de *CFTR* est complexé avec une nanoparticule à base d'acide polylactique-co-glycolique (PLGA).

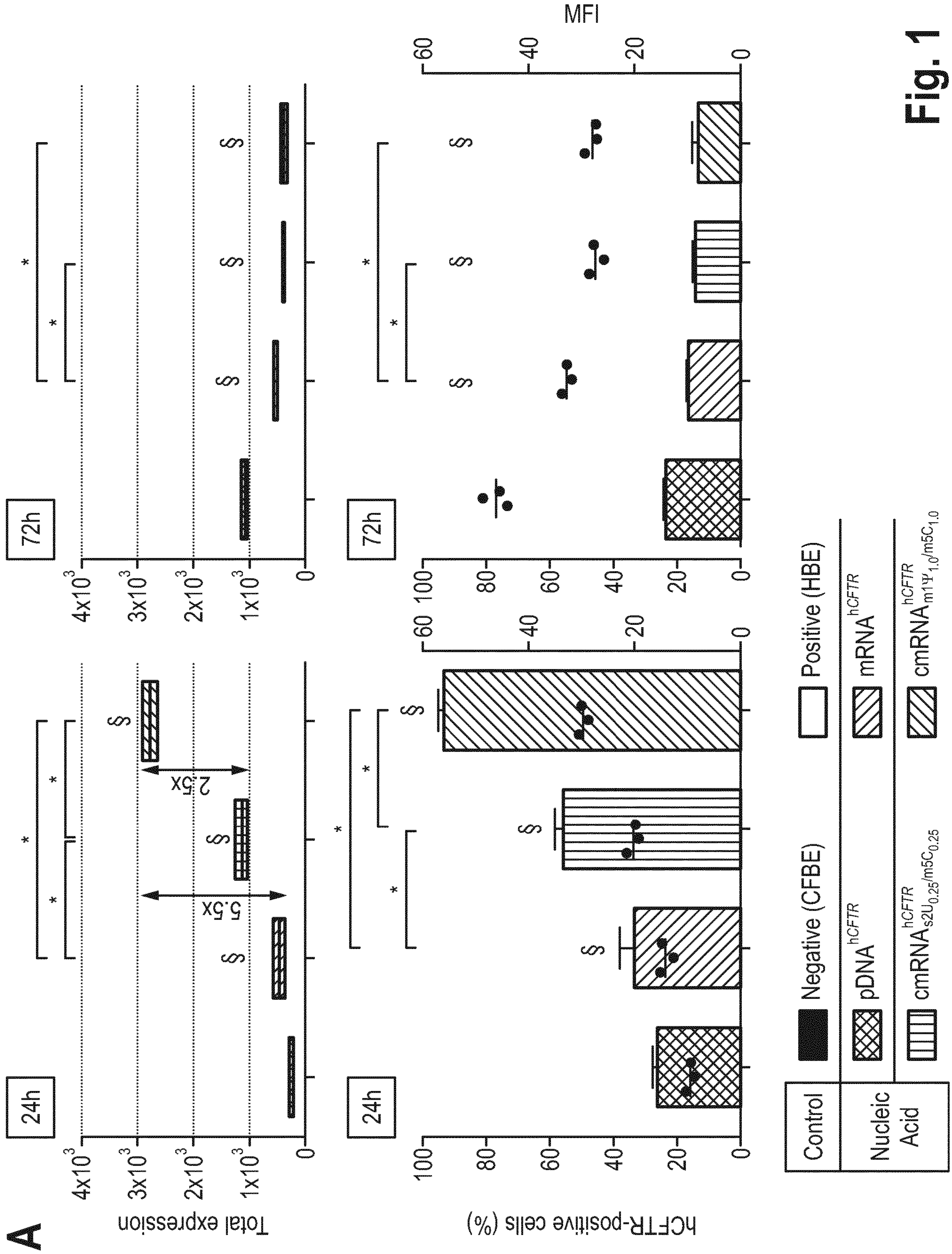
A
24h
72h
Total expression
4x10^3
3x10^3
2x10^3
1x10^3
0
5.5x
2.5x
hCFTR-positive cells (%)
100
80
60
40
20
MFI
Control
Negative (CFBE)
Positive (HBE)
Nucleic Acid
pDNA^hCFTR
mRNA^hCFTR
cmRNA^hCFTR s2U0.25/m5C0.25
cmRNA^hCFTR m1Ψ1.0/m5C1.0

Fig. 1

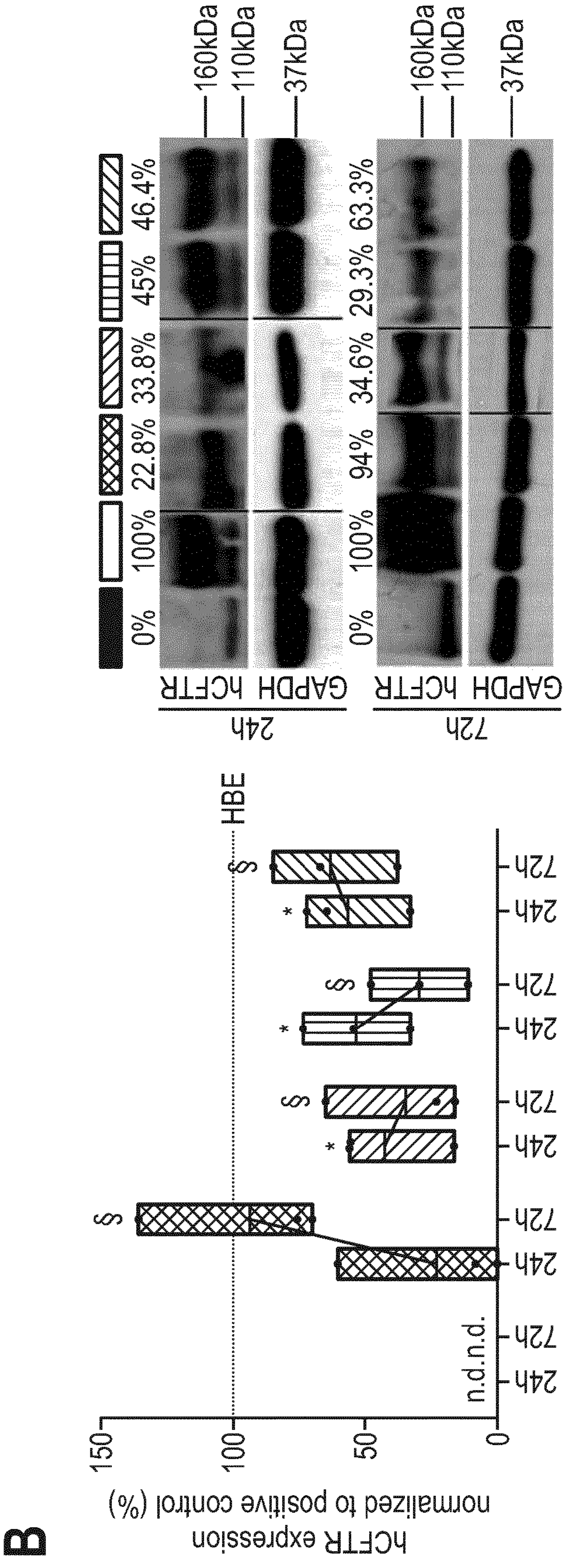
B
hCFTR expression
normalized to positive control (%)
150
100
50
0
HBE
n.d.
n.d.
24h
72h
§
*
0%
100%
22.8%
33.8%
45%
46.4%
94%
34.6%
29.3%
63.3%
hCFTR
GAPDH
160kDa
110kDa
37kDa


Fig. 1

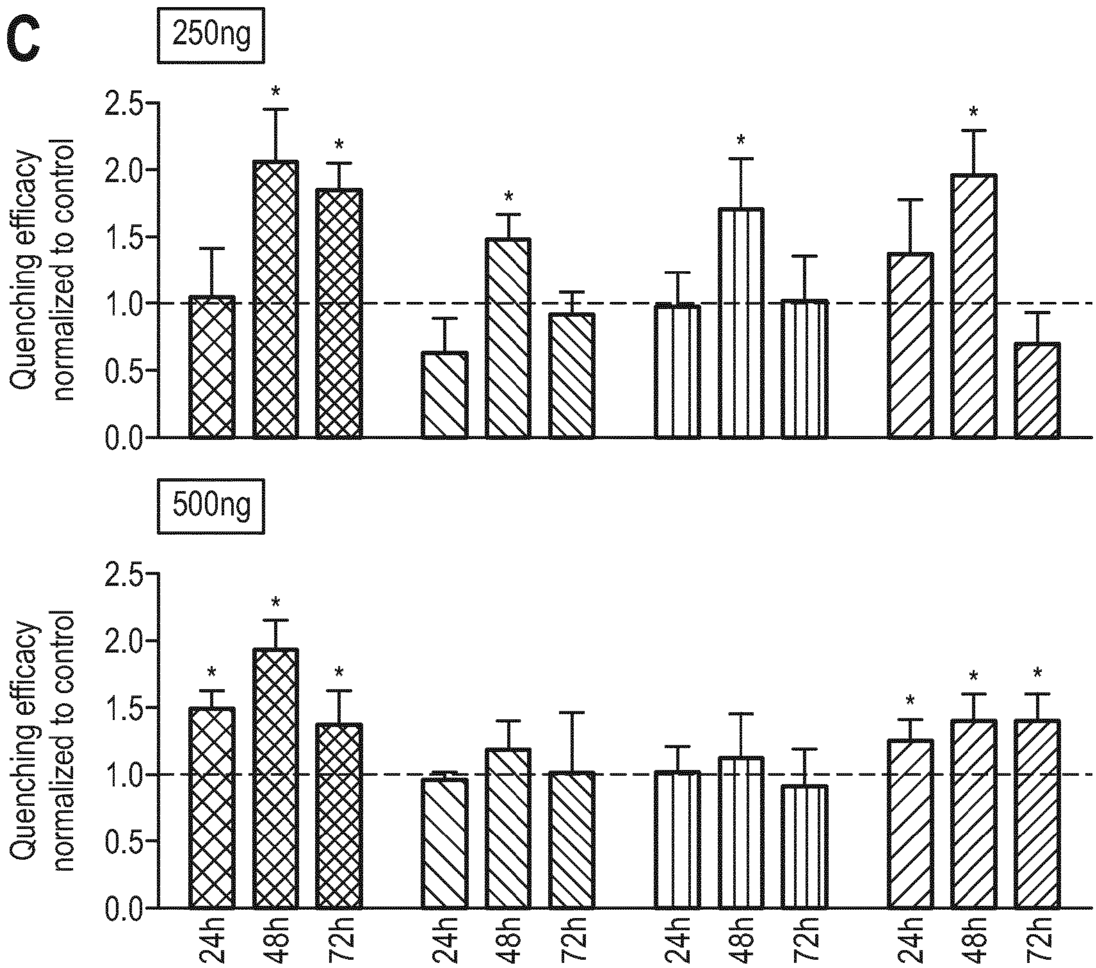
C
250ng
Quenching efficacy normalized to control
2.5
2.0
1.5
1.0
0.5
0.0
*
500ng
24h
48h
72h

Fig. 1

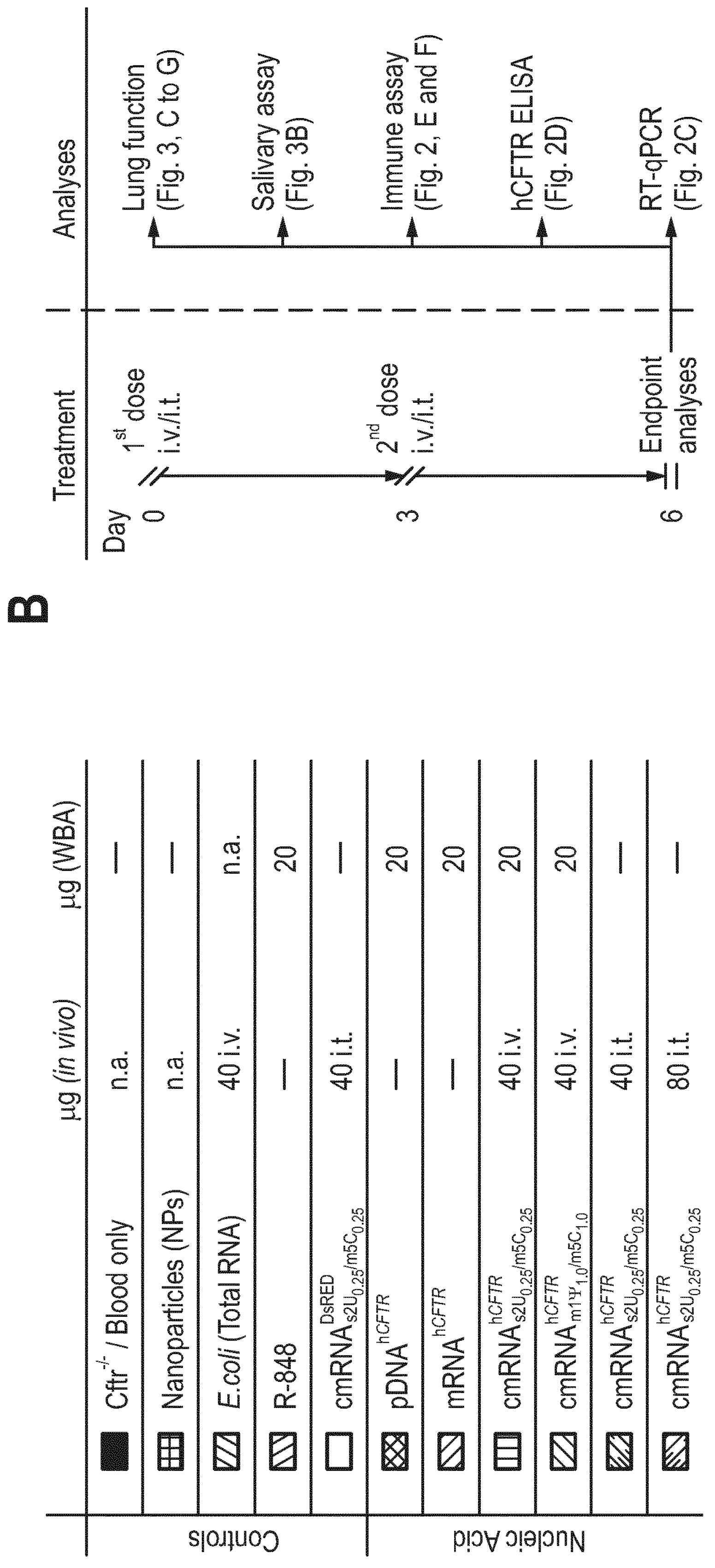
A
Controls
Nucleic Acid
µg (in vivo)
µg (WBA)
Cftr-/- / Blood only
n.a.
—
Nanoparticles (NPs)
n.a.
—
E.coli (Total RNA)
40 i.v.
n.a.
R-848
—
20
cmRNA DsRED s2U0.25/m5C0.25
40 i.t.
—
pDNA hCFTR
—
20
mRNA hCFTR
—
20
cmRNA hCFTR s2U0.25/m5C0.25
40 i.v.
20
cmRNA hCFTR m1Ψ1.0/m5C1.0
40 i.v.
20
cmRNA hCFTR s2U0.25/m5C0.25
40 i.t.
—
cmRNA hCFTR s2U0.25/m5C0.25
80 i.t.
—
B
Day
Treatment
Analyses
0
1st dose
i.v./i.t.
3
2nd dose
i.v./i.t.
6
Endpoint
analyses
Lung function
(Fig. 3, C to G)
Salivary assay
(Fig. 3B)
Immune assay
(Fig. 2, E and F)
hCFTR ELISA
(Fig. 2D)
RT-qPCR
(Fig. 2C)

Fig. 2

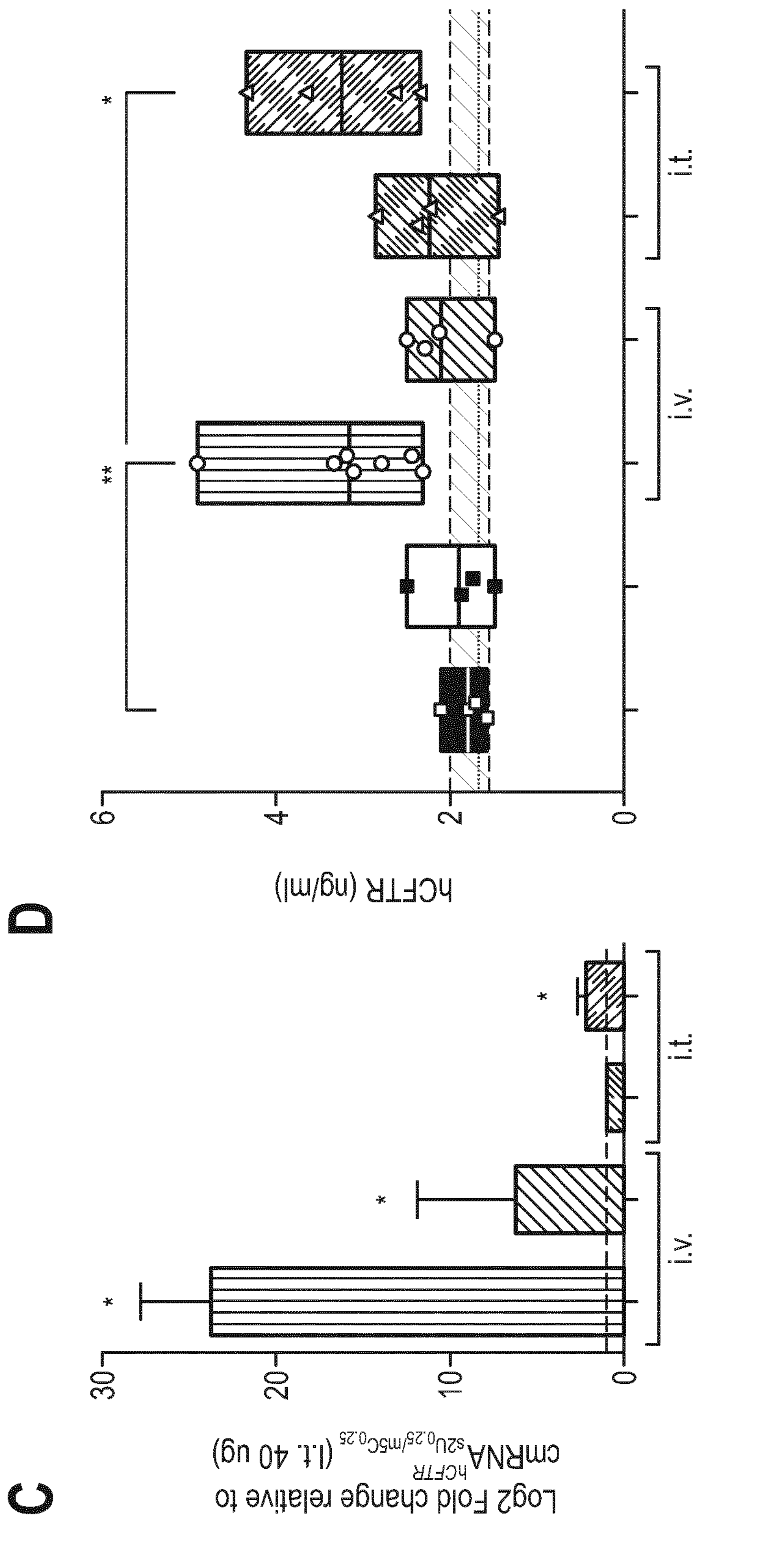
C
Log2 Fold change relative to cmRNA$^{hCFTR}_{s2U0.25/m5C0.25}$ (i.t. 40 ug)
0
10
20
30
*
*
*
i.v.
i.t.
D
hCFTR (ng/ml)
0
2
4
6
**
*
i.v.
i.t.

Fig. 2

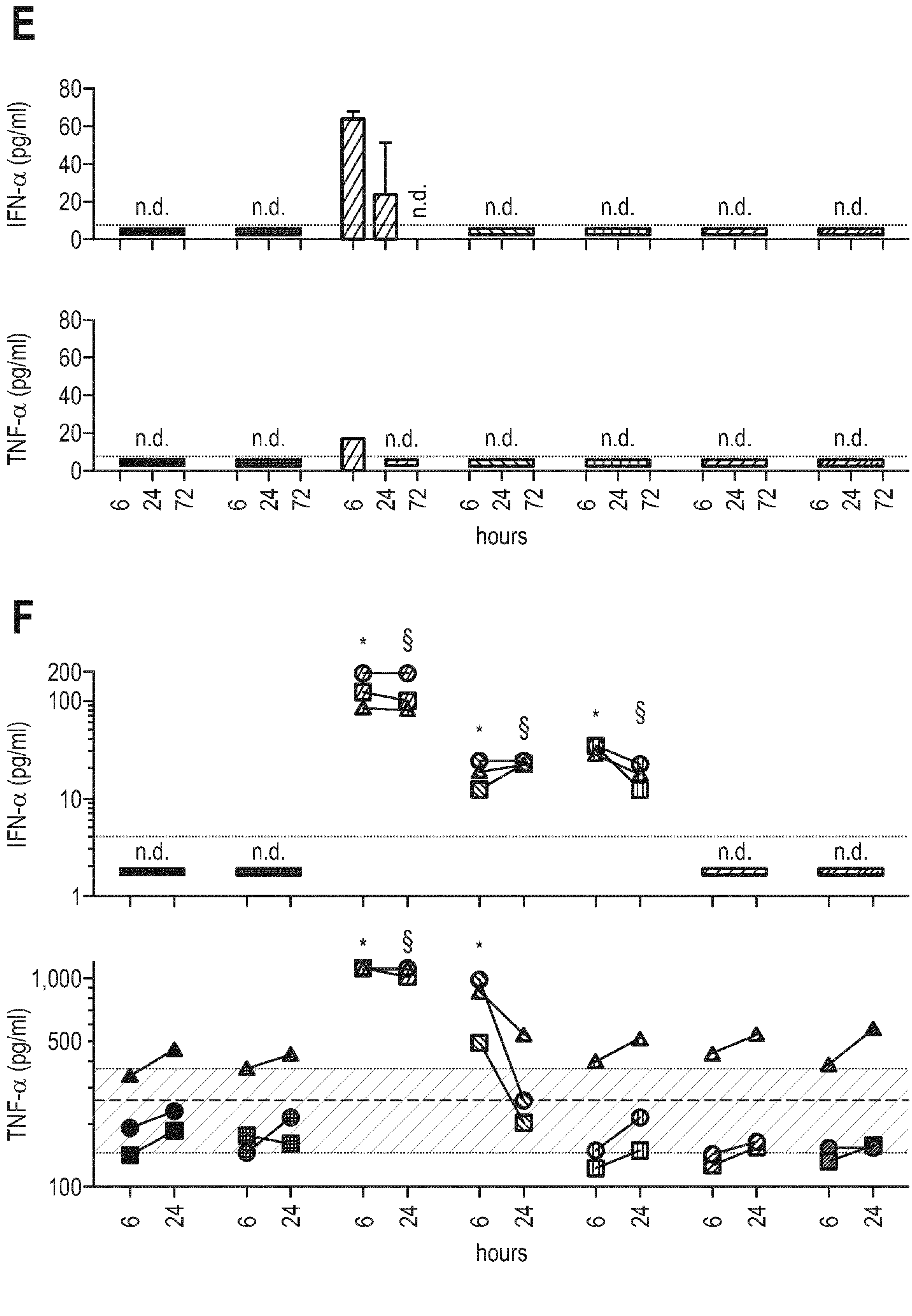


Fig. 2

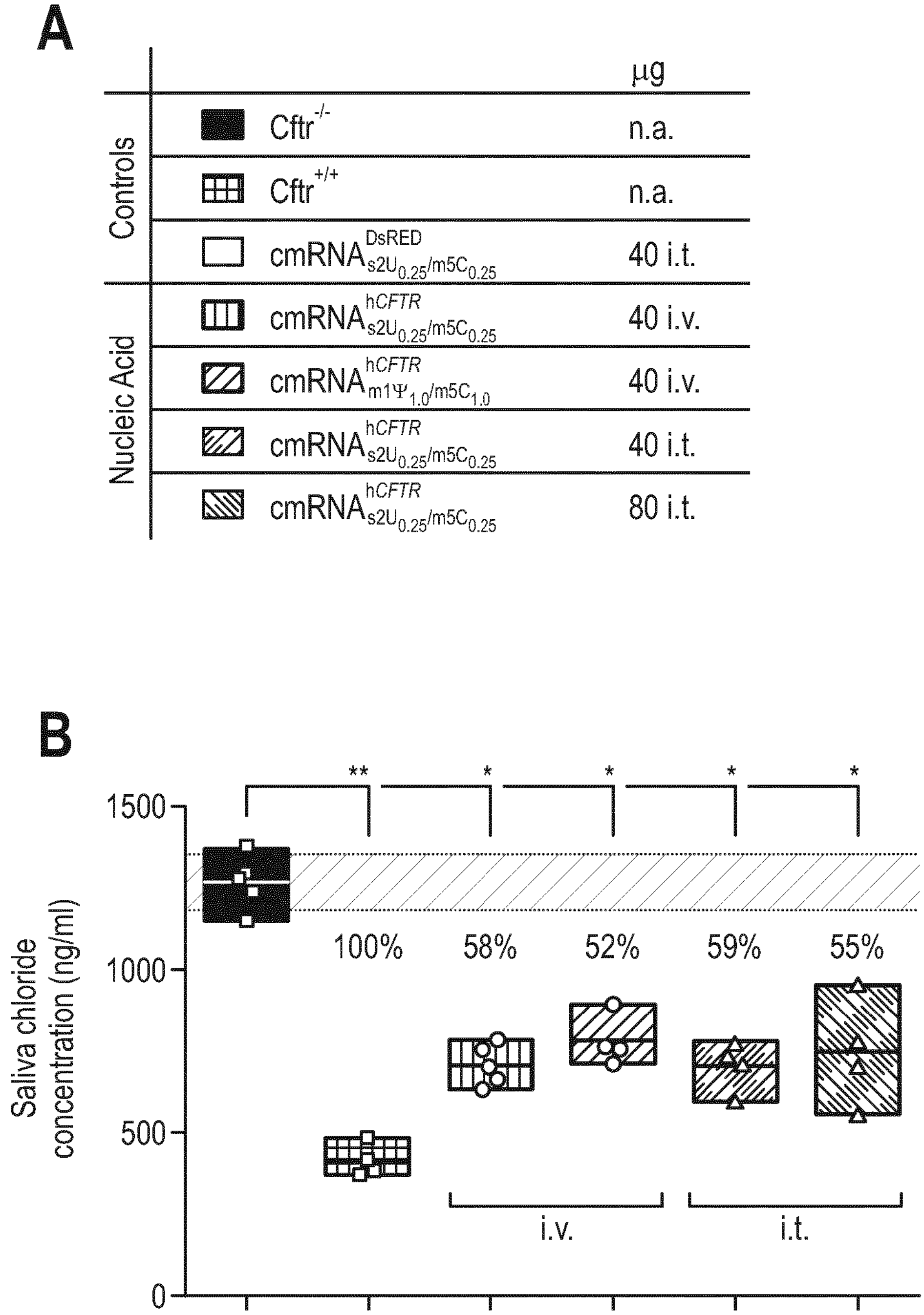
A
μg
Controls
Cftr-/-
n.a.
Cftr+/+
n.a.
cmRNA DsRED s2U0.25/m5C0.25
40 i.t.
Nucleic Acid
cmRNA hCFTR s2U0.25/m5C0.25
40 i.v.
cmRNA hCFTR m1Ψ1.0/m5C1.0
40 i.v.
cmRNA hCFTR s2U0.25/m5C0.25
40 i.t.
cmRNA hCFTR s2U0.25/m5C0.25
80 i.t.
B
Saliva chloride concentration (ng/ml)
1500
1000
500
0
**
*
*
*
*
100%
58%
52%
59%
55%
i.v.
i.t.

Fig. 3

C

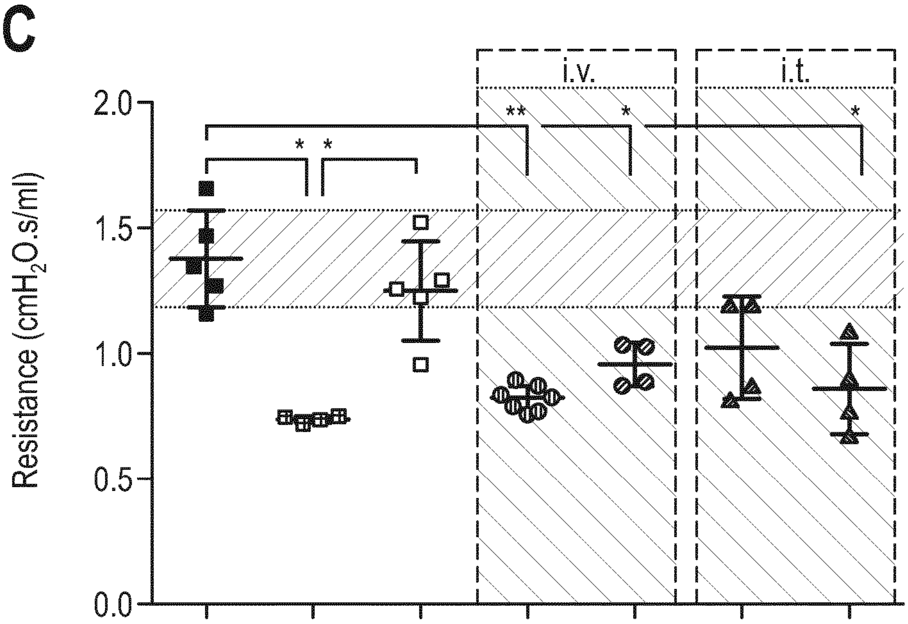
i.v.
i.t.
2.0
1.5
1.0
0.5
0.0
Resistance (cmH2O.s/ml)
** * *
* *

D

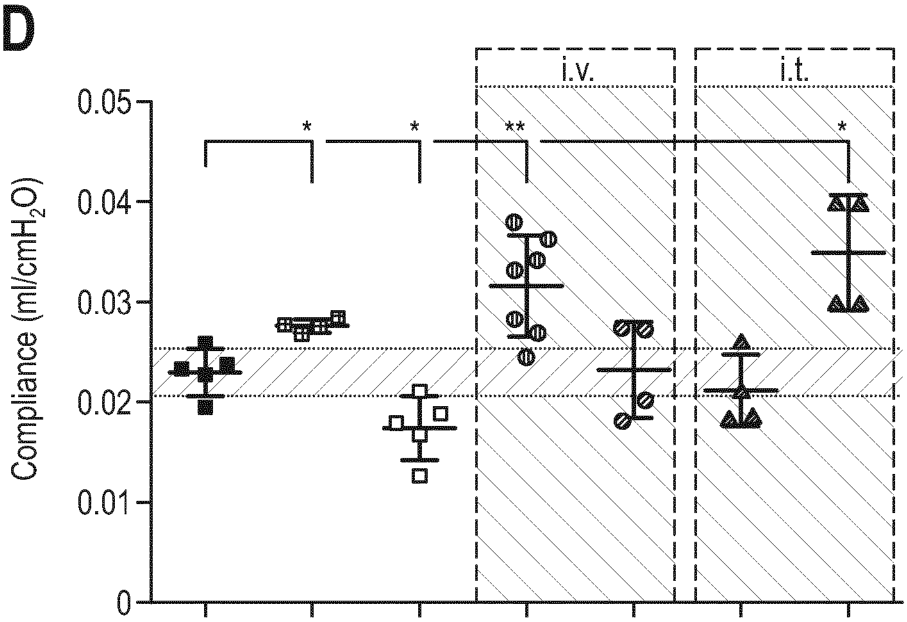
i.v.
i.t.
0.05
0.04
0.03
0.02
0.01
0
Compliance (ml/cmH2O)
* * ** *

Fig. 3

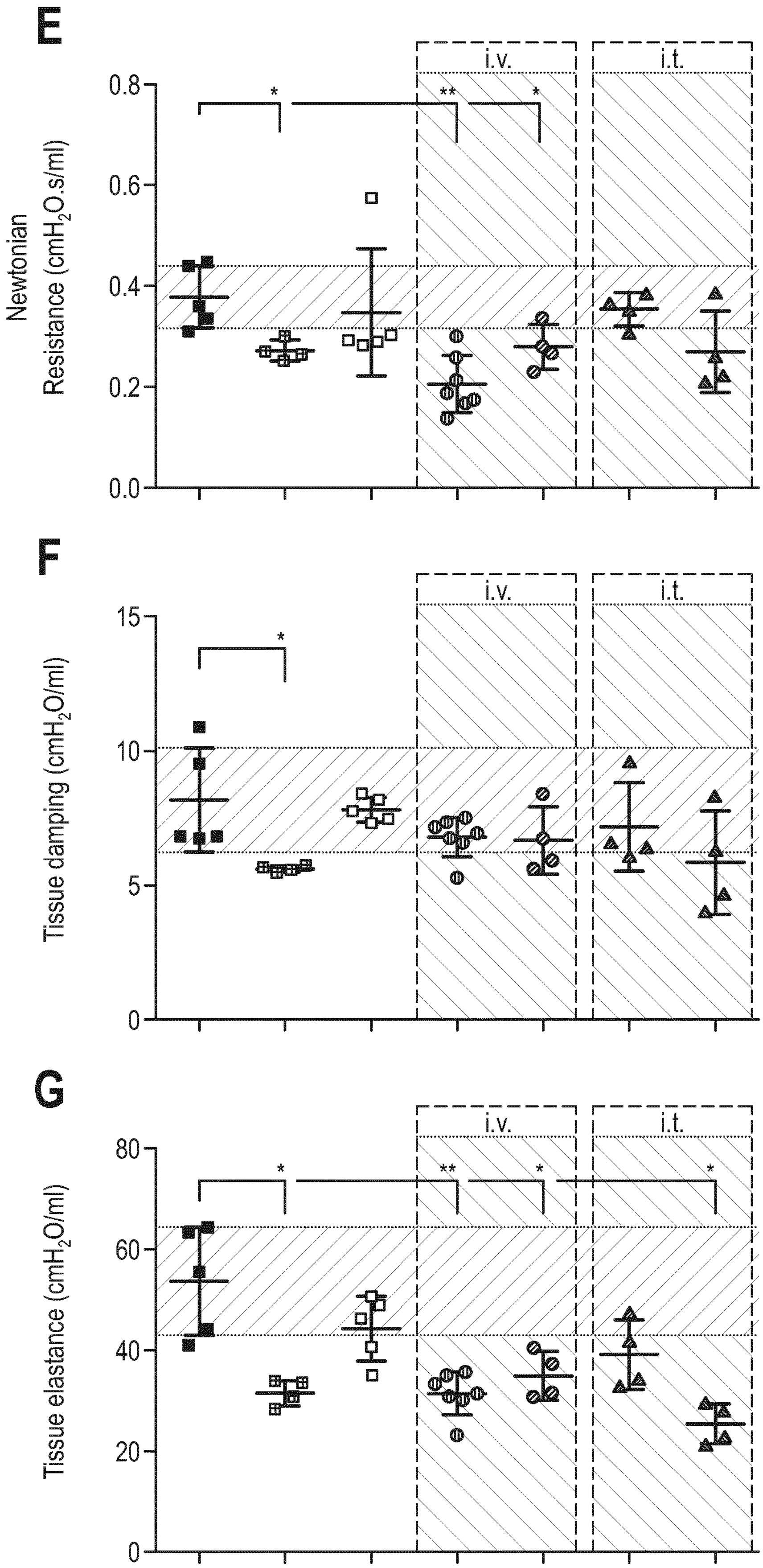
E
i.v.
i.t.
Newtonian Resistance (cmH2O.s/ml)
0.8
0.6
0.4
0.2
0.0
*
**
*
F
Tissue damping (cmH2O/ml)
15
10
5
0
G
Tissue elastance (cmH2O/ml)
80
60
40
20
0

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015052133 A
- WO 2011012316 A

### Non-patent literature cited in the description


- **KORMANN et al.** Expression of therapeutic proteins after delivery of chemically modified mRNA in mice. *Letters to Nature Biotechnology,* 2011, 1-6
- **MAHINY et al.** In vivo genome editing using nuclease-encoding mRNA corrects SP-B deficiency. *Nat. Biotechnology,* 2015, vol. 33 (6), 584-586
- **BANGEL-RULAND et al.** Cystic fibrosis transmembrane conductance regulator-mRNA delivery: a novel alternative for cystic fibrosis gene therapy. *J. Gene, Med.,* 2013, vol. 15, 414-426
- **KOLTE et al.** PEGylated composite nanoparticles of PLGA and polyethylenimine for safe and efficient delivery of pDNA to lungs. *Int. J. Pharm.,* 2017, vol. 524, 382-396
- **DANHIER et al.** PLGA-based nanoparticles: An overview of biomedical applications. *Journal of Controlled Release,* 2012, vol. 161 (2), 505-522
- **SAMBROOK ; RUSSELL.** Molecular Cloning: A Laboratory Manual. CSH Press, 2001
- Methods in Yeast Genetics, A Laboratory Course Manual. Cold Spring Harbor Laboratory Press, 1990